# EUROPEAN PATENT SPECIFICATION

(11) **EP 3 318 625 B1**
(45) Date of publication and mention of the grant of the patent: **08.01.2020**
(21) Application number: 17200484.8
(22) Date of filing: 08.11.2017
(51) Int. Cl.: C12N 1/20, A23C 9/123, A23K 10/18, A23L 33/135, C12R 1/225, C12R 1/46

(54) **A LACTIC ACID BACTERIA STRAIN**
MILCHSÄUREBAKTERIENSTAMM
SOUCHE DE BACTÉRIE LACTIQUE

(30) Priority: 08.11.2016 FI 20165837
(43) Date of publication of application: 09.05.2018
(73) Proprietor: Innolact Group Oy, 70210 Kuopio (FI)
(72) Inventor: HEINONEN, Jussi, 70210 Kuopio (FI); TAPAILA, Matti, 14140 Jokimaa (FI); VON WRIGHT, Atte, 70210 Kuopio (FI)
(74) Representative: Berggren Oy, Helsinki & Oulu

(56) References cited:
- WO-A2-2005/060708
- US-A1- 2015 017 208
- DATABASE EMBL [Online] 12 May 2009 (2009-05-12), "Lactococcus lactis subsp. cremoris gene for 16S rRNA, partial sequence, strain: O-114", XP002779193, Database accession no. AB469873 -& S. ITOI ET AL: "Phenotypic variation in Lactococcus lactis subsp. lactis isolates derived from intestinal tracts of marine and freshwater fish", JOURNAL OF APPLIED MICROBIOLOGY., vol. 107, no. 3, 1 September 2009 (2009-09-01), pages 867-874, XP055459935, GB ISSN: 1364-5072, DOI: 10.1111/j.1365-2672.2009.04266.x
- DATABASE EMBL [Online] 23 March 2009 (2009-03-23), "Lactococcus lactis subsp. cremoris strain IMAU40132 ribosomal RNA gene, partial sequence.", XP002779194, Database accession no. FJ749400 -& SUN ZHIHONG ET AL: "Identification and characterization of the dominant lactic acid bacteria from kurut: the naturally fermented yak milk in Qinghai, China.", THE JOURNAL OF GENERAL AND APPLIED MICROBIOLOGY FEB 2010, vol. 56, no. 1, February 2010 (2010-02), pages 1-10, XP002779195, ISSN: 0022-1260
- Anton Pallin: "Lactobacillus in the gastrointestinal tract of dog and wolf", , 1 January 2012 (2012-01-01), XP055459769, Retrieved from the Internet: URL:https://stud.epsilon.slu.se/4713/1/pal lin_a_120823.pdf.pdf [retrieved on 2018-03-15]
- J. KILLER ET AL: "Lactobacillus rodentium sp. nov., from the digestive tract of wild rodents", INTERNATIONAL JOURNAL OF SYSTEMATIC AND EVOLUTIONARY MICROBIOLOGY, vol. 64, no. Pt 5, 29 January 2014 (2014-01-29), pages 1526-1533, XP055459904, ISSN: 1466-5026, DOI: 10.1099/ijs.0.054924-0

### SEQUENCE LISTING

<110> Innolact Oy
<120> A lactic acid bacteria strain
<130> BP210683
<160> 7
<170> PatentIn version 3.5
<210> 1
   <211> 940
   <212> DNA
   <213> Lactococcus lactis ssp. cremoris
<400> 1
   gcttgcacca atttgaagag cagcgaacgg gtgagtaacg cgtggggaat ctgcctttga        60
   gcgggggaca acatttggaa acgaatgcta ataccgcata ataactttaa acataagttt       120
   taagtttgaa agatgcaatt gcatcactca aagatgatcc cgcgttgtat tagctagttg       180
   gtgaggtaaa ggctcaccaa ggcgatgata catagccgac ctgagagggt gatcggccac       240
   attgggactg agacacggcc caaactccta cgggaggcag cagtagggaa tcttcggcaa       300
   tggacgaaag tctgaccgag caacgccgcg tgagtgaaga aggttttcgg atcgtaaaac       360
   tctgttggta gagaagaacg ttggtgagag tggaaagctc atcaagtgac ggtaactacc       420
   cagaaaggga cggctaacta cgtgccagca gccgcggtaa tacgtaggtc ccgagcgttg       480
   tccggattta ttgggcgtaa agcgagcgca ggtggtttat taagtctggt gtaaaaggca       540
   gtggctcaac cattgtatgc attggaaact ggtagacttg agtgcaggag aggagagtgg       600
   aattccatgt gtagcggtga aatgcgtaga tatatggagg aacaccggtg gcgaaagcgg       660
   ctctctggcc tgtaactgac actgaggctc gaaagcgtgg ggagcaaaca ggattagata       720
   ccctggtagt ccacgccgta aacgatgagt gctagatgta gggagctata agttctctgt       780
   atcgcagcta acgcaataag cactccgcct ggggagtacg accgcaaggt tgaaactcaa       840
   aggaattgac gggggcccgc acaagcggtg gagcatgtgg tttaattcga agcaacgcga       900
   agaaccttac caggtcttga catactcgtg ctattcctag                             940
<210> 2
   <211> 706
   <212> DNA
   <213> Lactobacillus rodentium
<400> 2
   tttggtgctt gcaccaaatg aaaccagata caagcgagcg gcgaacgggt gagtaacacg        60
   tgggtaacct gcccaagaga ctgggataac acctggaaac agatgctaat accggataac       120
   aactctagac acatgtctgg agcttgaaag atgggttttc tatcactctt ggatggacct       180
   gcggtgcatt agctagttgg taaggtaacg gcttaccaag gcaatgatgc atagccgagt      240
   tgagagactg atcggccaca ttgggactga gacacggccc aaactcctac gggaggcagc      300
   agtagggaat cttccacaat ggacgcaagt ctgatggagc aacgccgcgt gagtgaagaa      360
   gggtttcggc tcgtaaagct ctgttgttgg tgaagaatga tagaggtagt aactggcctt      420
   tatttgacgg taatcaacca gaaagtcacg gctaactacg tgccagcagc cgcggtaata      480
   cgtaggtggc aagcgttgtc cggatttatt gggcgtaaag cgagcgcagg cggtttaata      540
   agtctgatgt gaaagccttc ggctcaaccg aagaattgca tcagaaactg ttaaacttga      600
   gtgcagaaga ggagagtgga actccatgtg tagcggtgga atgcgtagat atatggaaga      660
   acaccagtgg cgaaggcggc tctctggtct gcaactgacg ctgagg                     706
<210> 3
   <211> 959
   <212> DNA
   <213> Lactobacillus rodentium
<400> 3
   ttttggtgct tgcaccaaat gaaaccagat acaagcgagc ggcgaacggg tgagtaacac       60
   gtgggtaacc tgcccaagag actgggataa cacctggaaa cagatgctaa taccggataa      120
   caactctaga cacatgtctg gagcttgaaa gatgggtttt ctatcactct tggatggacc      180
   tgcggtgcat tagctagttg gtaaggtaac ggcttaccaa ggcaatgatg catagccgag      240
   ttgagagact gatcggccac attgggactg agacacggcc caaactccta cgggaggcag      300
   cagtagggaa tcttccacaa tggacgcaag tctgatggag caacgccgcg tgagtgaaga      360
   agggtttcgg ctcgtaaagc tctgttgttg gtgaagaatg atagaggtag taactggcct      420
   ttatttgacg gtaatcaacc agaaagtcac ggctaactac gtgccagcag ccgcggtaat      480
   acgtaggtgg caagcgttgt ccggatttat tgggcgtaaa gcgagcgcag gcggtttaat      540
   aagtctgatg tgaaagcctt cggctcaacc gaagaattgc atcagaaact gttaaacttg      600
   agtgcagaag aggagagtgg aactccatgt gtagcggtgg aatgcgtaga tatatggaag      660
   aacaccagtg gcgaaggcgg ctctctggtc tgcaactgac gctgaggctc gaaagcatgg      720
   gtagcgaaca ggattagata ccctggtagt ccatgccgta aacgatgagt gctaagtgtt      780
   gggaggtttc cgcctctcag tgctgcagct aacgcattaa gcactccgcc tggggagtac      840
   gaccgcaagg ttgaaactca aaggaattga cgggggcccg cacaagcggt ggagcatgtg      900
   gtttaattcg aagcaacgcg aagaacctta ccaggtcttg acatccagtg caatcctaa       959
<210> 4
   <211> 958
   <212> DNA
   <213> Lactobacillus rodentium
<400> 4
   ttttggtgct tgcaccaaat gaaaccagat acaagcgagc ggcgaacggg tgagtaacac      60
   gtgggtaacc tgcccaagag actgggataa cacctggaaa cagatgctaa taccggataa     120
   caactctaga cacatgtctg gagcttgaaa gatgggtttt ctatcactct tggatggacc     180
   tgcggtgcat tagctagttg gtaaggtaac ggcttaccaa ggcaatgatg catagccgag     240
   ttgagagact gatcggccac attgggactg agacacggcc caaactccta cgggaggcag     300
   cagtagggaa tcttccacaa tggacgcaag tctgatggag caacgccgcg tgagtgaaga     360
   agggtttcgg ctcgtaaagc tctgttgttg gtgaagaatg atagaggtag taactggcct     420
   ttatttgacg gtaatcaacc agaaagtcac ggctaactac gtgccagcag ccgcggtaat     480
   acgtaggtgg caagcgttgt ccggatttat tgggcgtaaa gcgagcgcag gcggtttaat     540
   aagtctgatg tgaaagcctt cggctcaacc gaagaattgc atcagaaact gttaaacttg     600
   agtgcagaag aggagagtgg aactccatgt gtagcggtgg aatgcgtaga tatatggaag     660
   aacaccagtg gcgaaggcgg ctctctggtc tgcaactgac gctgaggctc gaaagcatgg     720
   gtagcgaaca ggattagata ccctggtagt ccatgccgta aacgatgagt gctaagtgtt     780
   gggaggtttc cgcctctcag tgctgcagct aacgcattaa gcactccgcc tggggagtac     840
   gaccgcaagg ttgaaactca aaggaattga cgggggcccg cacaagcggt ggagcatgtg     900
   gtttaattcg aagcaacgcg aagaacctta ccaggtcttg acatccagtg caatccta       958
<210> 5
   <211> 929
   <212> DNA
   <213> Lactobacillus rodentium
<400> 5
   tacaagcgag cggcgaacgg gtgagtaaca cgtgggtaac ctgcccaaga gactgggata        60
   acacctggaa acagatgcta ataccggata acaactctag acacatgtct ggagcttgaa       120
   agatgggttt tctatcactc ttggatggac ctgcggtgca ttagctagtt ggtaaggtaa       180
   cggcttacca aggcaatgat gcatagccga gttgagagac tgatcggcca cattgggact       240
   gagacacggc ccaaactcct acgggaggca gcagtaggga atcttccaca atggacgcaa       300
   gtctgatgga gcaacgccgc gtgagtgaag aagggtttcg gctcgtaaag ctctgttgtt       360
   ggtgaagaat gatagaggta gtaactggcc tttatttgac ggtaatcaac cagaaagtca       420
   cggctaacta cgtgccagca gccgcggtaa tacgtaggtg gcaagcgttg tccggattta       480
   ttgggcgtaa agcgagcgca ggcggtttaa taagtctgat gtgaaagcct tcggctcaac       540
   cgaagaattg catcagaaac tgttaaactt gagtgcagaa gaggagagtg gaactccatg       600
   tgtagcggtg gaatgcgtag atatatggaa gaacaccagt ggcgaaggcg gctctctggt       660
   ctgcaactga cgctgaggct cgaaagcatg ggtagcgaac aggattagat accctggtag       720
   tccatgccgt aaacgatgag tgctaagtgt tgggaggttt ccgcctctca gtgctgcagc       780
   taacgcatta agcactccgc ctggggagta cgaccgcaag gttgaaactc aaaggaattg       840
   acgggggccc gcacaagcgg tggagcatgt ggtttaattc gaagcaacgc gaagaacctt       900
   accaggtctt gacatccagt gcaatccta                                         929
<210> 6
   <211> 20
   <212> DNA
   <213> artificial
<220>
   <223> primer
<400> 6
   agagtttgat cctggctcag 20
<210> 7
   <211> 21
   <212> DNA
   <213> artificial
<220>
   <223> primer
<400> 7
   acggctacct tgttacgact t 21

## Description

### Field of the invention

The present application relates to a lactic acid bacterial strain and a biologically active culture for probiotic use and to a food composition or a nutritional supplement comprising said lactic acid bacterial strain. In addition the application relates to methods of using said strain or culture in maintenance of wellbeing and health and prevention of undesired conditions.

### Background

The significance of probiotics for human intestine, wellbeing and balance of immune system is well known. Also their effect on human metabolism, including overcoming and prevention of diseases, protection of human body against harmful microbiota and even providing positive effects on mental health has been reported. Every mammalian has specific microbiota in their body system.

For dogs powder and tablet form probiotics are available in the market. These are, however, manufactured from lactic acid bacteria of human or dog origin and they are not very efficient.

Master's thesis by Anton Pallin (Department of Microbiology, Master's thesis, Examenarbete/Sveriges lantbruksuniversitet, Institutionen för mikrobiologi: 201: 10, IS-SA 1101-8151; Uppsala 2012) discloses lactobacilli derived from the gut of wolves. *L. reuteri* strains are deemed potential probiotic but they do not show suitable mucus binding capacity.

Thus there is a need for more effective probiotics for canines, especially for dogs.

### Summary

An aim of the invention is to provide a lactic acid bacteria strain or a culture for probiotic use and methods for using such strain or culture.

One object of the invention is a lactic acid bacteria strain for probiotic use. Said strain is characterized by 16 S RNA sequence defined by SEQ ID NO: 1 and having the deposit number VTT E-173549.

Another object of the invention is a biologically active culture comprising strain of at least one lactic acid bacteria. Said strain is characterized by 16 S RNA sequence defined by SEQ ID NO: 1 and having the deposit number VTT E-173549.

Another object of the invention is a food composition or a nutritional supplement for canine. Characteristic to said composition or supplement is that it comprises one or more of strain(s) or culture(s) described here.

Described herein is a method for maintaining or improving microbial flora in gastrointestinal tract. Said method comprises administering to the subject an effective amount of one or more of strain(s) or culture(s) described here.

Also described is a method for maintaining or improving wellbeing of immune system, or gastrointestinal tract or dental health or any combination thereof. Said method comprises administering to the subject an effective amount of one or more of strain(s) or culture(s) described here.

Also described is a method for treating or preventing diarrhea or Inflammatory bowel disease oe giardiasis or atopic dermatitis or food sensitivities or any combination thereof. Said method comprises administering to the subject an effective amount of one or more of strain(s) or culture(s) described here.

Further objects of the invention are uses of the lactic acid bacteria strain described above.

Still further object of the invention is a method for preparation of the milk based composition described here. Said method comprising the steps of:
a) fat standardization; and
b) protein-standardization; and
c) homogenization; and
d) pasteurization; and
e) cooling; and
f) inoculation with starter cultures; and
g) incubation and cooling; and
h) stirring; and
i) pasteurization; and
j) inoculation with the strain or a culture described above and
k) packing.

The main embodiments are characterized in the independent claims. Various embodiments are disclosed in the dependent claims and the description. The features recited in dependent claims and in the embodiments are mutually freely combinable unless otherwise explicitly stated.

### Short description of the Figure

**Figure 1** shows Results of biochemical characterization tests of lactic acid bacteria and Bergey's manual biochemical characterization data of closest matching species in 16S rRNA gene sequence data comparison and biochemical characterization data available for Lactobacillus rodentium. The divergent results from lactic acid bacteria are indicated by parenthesis. ¹De Vos P, Garrity GM, Jones D, Krieg NR, Ludwig W, Rainey FA, Schleifer K-H & Whitman WB, 2009. Bergey's manual of systematic bacteriology, 2nd edition, volume 3 The Firmicutes. Springer, Athens, USA,1450 pages; ²Killer J, Havlik J, Vlkova E, Rada V, Pechar R, Benada O, Kopecny J, kofronova O & Sechocova H, 2014. Lactobacillus rodentium sp. nov., from the digestive tract of wild rodents. Int J Syst Evol Microbiol 64(5), 1526-1533. "-/-" or "+" indicates that the result was not clear.

### Deposits

The following strain depositions according to the Budapest Treaty on the International Recognition of Deposit of Microorganisms for the Purposes of Patent Procedure were made at the VTT Culture Collection, P.O. Box 1000 (Tietotie 2), FI-02044 VTT, Finland on 2 November 2017:
*Lactococcus lactis ssp. cremoris* 11809_6 assigned accession number VTT E-173549;
*Lactobacillus rodentium* 11822_3 assigned accession number VTT E-173545;
*Lactobacillus rodentium* 11822_4 assigned accession number VTT E-173546;
*Lactobacillus rodentium* 11822_5 assigned accession number VTT E-173547; and
*Lactobacillus rodentium* 11822_8 assigned accession number VTT E-173548.

### Detailed description

The aim of the present study was to develop probiotic product for dogs to add their wellbeing in many ways. All canines have similar type microbiota in their intestinal, though individual variation occurs mostly because of the environmental facts (for example nutrition). The wolf, (*Canis lupus*), member of the canine family, is biologically same species as dog, but wolves live in much more demanding environments requiring robust intestinal health, which gives their intestinal microbiota special properties. The inventors have surprisingly found that it is possible to use probiotic microbes of wolf intestine to enhance pet dogs wellbeing.

Both powder and tablet form probiotics for dogs are available in the market but they are usually manufactured from human or dog origin lactic acid bacteria. The present inventors have surprisingly found that microbes derived from wolf gastrointestinal tract are more effective. It is believed that this is because such microbes are adapted to live in the wolf intestinal tract in very demanding environmental and nutritional conditions.

In general the strains, cultures, compositions methods and uses described herein are especially suitable for canines. Canines, as used herein refer to all members of the biological family Canidae, which is a lineage of carnivorans that includes domestic dogs, wolves, foxes, jackals, dingoes, and many other extant and extinct dog-like mammals. A member of this family may be also called a canid. The canines may refer to genus *Canis,* such as *Canis lupus,* more particularly *Canis lupus familiaris.* Therefore one specific example of a canine or a canid is a dog, such as a domestic dog.

One embodiment provides a lactic acid bacteria strain for probiotic use characterized by 16 S RNA sequence defined by SEQ ID NO: 1, SEQ ID NO: 2, SEQ ID NO: 3, SEQ ID NO: 4 or SEQ ID NO: 5. 16 S RNA sequence carried by a strain is unique to said strain and thus suitable for exact identification. Any known methods may be used for detecting the RNA sequence, such as Northern blotting, sequencing techniques, and the like but sequencing is needed to ensure the result. Strain specific PCR can also be developed, and additional techniques like Random Amplified DNA Polymorfism (RAPD) and Pulsed Field Gel Electrophoresis (PFGE) of genomic DNA can also be applied.

One embodiment provides a biologically active culture comprising strain of at least one lactic acid bacteria characterized by 16 S RNA sequence defined by SEQ ID NO: 1, SEQ ID NO: 2, SEQ ID NO: 3, SEQ ID NO: 4 or SEQ ID NO: 5

The biological activity as used herein refers especially to viability and adhesion properties of the strain or the culture. Expression "viable" means that the cells are alive and viable in the body, such as in the intestine, of a subject, for at least the standard intestinal transit time (usually 12 to 48 h). Expression "adhesion ability" means that the strain or culture is able to adhere to intestinal mucosa or to other desired target in the body of a subject. Good adherence prolongs the time that said strain can be active in the body of the subject. In this study the adherence to C2BBe1- enterocyte cells has been used to simulate *in vivo* adherence.

One embodiment provides said strain or said culture, wherein the strain has an ability to adhere to mucosa in gastrointestinal tract. Preferably the adherence is at least 80% of the adherence of commercial *L. rhamnosus GG* strain. Specifically the adhesion is evaluated by exposing C2BBe1 cells , incubated for 14 days, by the strains here described using *L. rhamnosus GG,* the commercial strain which is known for good adhesion ability, as positive control and M17 broth+0.5% glucose and MRS broth as negative controls and C2BBe1-medium as zero control. After the exposure, the medium was removed from the wells and cells were washed. Dilutions are prepared and samples are plated on suitable media and incubated and compared to controls.

One embodiment provides said strain or said culture, wherein the strain has an ability to use sugars as shown in Figure 1.

In one embodiment the strain or the culture comprising the strain is characterized by 16 S RNA sequence defined by SEQ ID NO: 1 (*Lactococcus lactis ssp. cremoris* 11809_6, deposited to the VTT Culture Collection under the accession number VTT E-173549), the ability to adhere the mucosa of gastrointestinal tract and the ability to use the sugars as follows:

**Table 1**

| Test/characteristics | Test | Lactic acid bacteria 11809_6 | Bergeys manual | Bergeys manual |
|---|---|---|---|---|
| | | | *Lactococcus lactis* ssp. *cremoris* | *Lactococcus lactis* ssp. *lactis* |
| Glycerol | API 50CH | - | NA | NA |
| Erythrotritol | API 50CH | - | NA | NA |
| D-Arabinose | API 50CH | - | NA | NA |
| L-Arabinose | API 50CH | - | NA | NA |
| D-xylose | API 50CH | + | NA | NA |
| L-xylose | API 50CH | - | NA | NA |
| D-Adonitol | API 50CH | - | NA | NA |
| Methyl-βD-Xylanopyroside | API 50CH | - | NA | NA |
| D-Glucose | API 50CH | + | NA | NA |
| D-Fructose | API 50CH | + | NA | NA |
| D-Mannose | API 50CH | + | NA | NA |
| L-Sorbose | API 50CH | - | NA | NA |
| L-Rhamnose | API 50CH | - | NA | NA |
| Dulcitol | API 50CH | - | NA | NA |
| Inositol | API 50CH | - | NA | NA |
| D-Mannitol | API 50CH | + | NA | NA |
| D-Sorbitol | API 50CH | - | NA | NA |
| Methyl-αD-Mannopyranoside | API 50CH | - | NA | NA |
| Methyl-αD-Glucopyranoside | API 50CH | - | NA | NA |
| N-Acetylglucosamine | API 50CH | + | NA | NA |
| Arbutin | API 50CH | + | NA | NA |
| Esculin ferric citrate | API 50CH | + | NA | NA |
| Salicin | API 50CH | + | NA | NA |
| D-Cellobiose | API 50CH | + | NA | NA |
| D-Lactose | API 50CH | + | + | + |
| D-Melibiose | API 50CH | - | - | - |
| D-Saccharose | API 50CH | - | NA | NA |
| D-Trehalose | API 50CH | + | NA | NA |
| Inulin | API 50CH | - | NA | NA |
| D-Melezitose | API 50CH | - | - | - |
| D-Raffinose | API 50CH | - | - | - |
| Glycogen | API 50CH | - | NA | NA |
| Xylitol | API 50CH | - | NA | NA |
| Gentiobiose | API 50CH | + | NA | NA |
| D-Turanose | API 50CH | - | NA | NA |
| D-Lyxose | API 50CH | - | NA | NA |
| D-Tagatose | API 50CH | - | NA | NA |
| D-Fucose | API 50CH | - | NA | NA |
| L-Fucose | API 50CH | - | NA | NA |
| D-Arabitol | API 50CH | - | NA | NA |
| L-Arabitol | API 50CH | - | NA | NA |
| Potassium 2-ketogluconate | API 50CH | - | NA | NA |
| Potassium 5-ketogluconate | API 50CH | - | NA | NA |

In a further embodiment said strain or culture is susceptible to Gentamicin, Kanamycin, Streptomycin, Tetracycline, Erythromycin, Clindamycin, Chloramphenicol, Ampicillin and Vancomycin analysed according to ISO10932:2010 (when suitable) standard with VetMIC Lact-1 and VetMIC Lact-2 plates (SVA National Veterinary Institute, Uppsala, Sweden) in anaerobic conditions at +37°C for 48 hours.

In one embodiment the strain or the culture comprising the strain is characterized by 16 S RNA sequence defined by SEQ ID NO: 2 (*Lactobacillus rodentium* 11822_3, deposited to the VTT Culture Collection under the accession number VTT E-173545), the ability to adhere the mucosa of gastrointestinal tract and the ability to use the sugars as follows

**Table 2.**

| Test/characteristics | Test | Lactic acid bacteria 11822_3 | Bergeys manual | Bergeys manual | Bergeys manual | Killer et al 2014 Lactobacillus rodentium |
|---|---|---|---|---|---|---|
| | | | *Lactobacillus jensenii* | *Lactobacillus gasserii* | *Lactobacillus johnsonii* | |
| Glycerol | API 50CH | - | NA | NA | NA | NA |
| Erythrotritol | API 50CH | - | NA | NA | NA | NA |
| D-Arabinose | API 50CH | - | - | NA | NA | NA |
| L-Arabinose | API 50CH | - | NA | NA | NA | NA |
| D-xylose | API 50CH | - | - | NA | NA | NA |
| L-xylose | API 50CH | - | NA | NA | NA | NA |
| D-Adonitol | API 50CH | - | NA | NA | NA | NA |
| Methyl-βD-Xylanopyroside | API 50CH | - | NA | NA | NA | NA |
| D-Glucose | API 50CH | + | NA | NA | NA | NA |
| D-Fructose | API 50CH | + | NA | NA | NA | NA |
| D-Mannose | API 50CH | + | NA | + | + | d |
| L-Sorbose | API 50CH | - | NA | NA | NA | NA |
| L-Rhamnose | API 50CH | - | NA | NA | NA | NA |
| Dulcitol | API 50CH | - | NA | NA | NA | NA |
| Inositol | API 50CH | - | NA | NA | NA | NA |
| D-Mannitol | API 50CH | - | d | - | - | NA |
| D-Sorbitol | API 50CH | - | - | NA | NA | NA |
| Methyl-αD-Mannopyranoside | API 50CH | - | NA | NA | NA | NA |
| Methyl-αD-Glucopyranoside | API 50CH | - | NA | NA | NA | NA |
| N-Acetylglucosamine | API 50CH | + | NA | NA | NA | d |
| Amygdalin | API 50CH | + | + | + | + | d |
| Esculin ferric citrate | API 50CH | + | + | NA | NA | d |
| Salicin | API 50CH | + | NA | + | + | d |
| D-Cellobiose | API 50CH | + | + | + | + | d |
| D-Maltose | API 50CH | + | NA | d | + | NA |
| D-Lactose | API 50CH | + | NA | d | d | + |
| D-Saccharose | API 50CH | + | + | + | + | NA |
| D-Trehalose | API 50CH | + | NA | d | d | NA |
| Inulin | API 50CH | - | NA | NA | NA | NA |
| D-Melezitose | API 50CH | - | - | NA | NA | NA |
| Amidon | API 50CH | - | NA | NA | NA | NA |
| Glycogen | API 50CH | - | NA | NA | NA | NA |
| Xylitol | API 50CH | - | NA | NA | NA | NA |
| Gentiobiose | API 50CH | + | NA | NA | NA | + |
| D-Turanose | API 50CH | - | NA | NA | NA | NA |
| D-Lyxose | API 50CH | - | NA | NA | NA | NA |
| D-Tagatose | API 50CH | - | NA | NA | NA | - |
| D-Fucose | API 50CH | - | NA | NA | NA | NA |
| L-Fucose | API 50CH | - | NA | NA | NA | NA |
| D-Arabitol | API 50CH | - | NA | NA | NA | NA |
| L-Arabitol | API 50CH | - | NA | NA | NA | NA |
| Potassium Gluconate | API 50CH | - | NA | NA | NA | NA |
| Potassium 2-ketogluconate | API 50CH | - | NA | NA | NA | NA |
| Potassium 5-ketogluconate | API 50CH | - | NA | NA | NA | NA |

In a further embodiment said strain or culture is susceptible to Gentamicin, Kanamycin, Streptomycin, Tetracycline, Erythromycin, Clindamycin, Chloramphenicol, Ampicillin and Vancomycin analysed according to ISO10932:2010 (when suitable) standard with VetMIC Lact-1 and VetMIC Lact-2 plates (SVA National Veterinary Institute, Uppsala, Sweden) in anaerobic conditions at +37°C for 48 hours.

In one embodiment the strain or the culture comprising the strain is characterized by 16 S RNA sequence defined by SEQ ID NO: 3 (*Lactobacillus rodentium* 11822_4, deposited to the VTT Culture Collection under the accession number VTT E-173546), the ability to adhere the mucosa of gastrointestinal tract and the ability to use the sugars as follows

**Table 3**

| Test/characteristics | Test | Lactic acid bacteria 11822_4 | Bergeys manual *Lactobacillus jensenii* | Bergeys manual *Lactobacillus gasseri* | Bergeys manual *Lactobacillus johnsonii* | Killer et al 2014 Lactobacillus rodentium |
|---|---|---|---|---|---|---|
| Glycerol | API 50CH | - | NA | NA | NA | NA |
| Erythrotritol | API 50CH | - | NA | NA | NA | NA |
| D-Arabinose | API 50CH | - | - | NA | NA | NA |
| L-Arabinose | API 50CH | - | NA | NA | NA | NA |
| D-xylose | API 50CH | - | - | NA | NA | NA |
| L-xylose | API 50CH | - | NA | NA | NA | NA |
| D-Adonitol | API 50CH | - | NA | NA | NA | NA |
| Methyl-βD-Xylanopyroside | API 50CH | - | NA | NA | NA | NA |
| D-Glucose | API 50CH | + | NA | NA | NA | NA |
| D-Fructose | API 50CH | + | NA | NA | NA | NA |
| D-Mannose | API 50CH | + | NA | + | + | d |
| L-Sorbose | API 50CH | - | NA | NA | NA | NA |
| L-Rhamnose | API 50CH | - | NA | NA | NA | NA |
| Dulcitol | API 50CH | - | NA | NA | NA | NA |
| Inositol | API 50CH | - | NA | NA | NA | NA |
| D-Mannitol | API 50CH | - | d | - | - | NA |
| D-Sorbitol | API 50CH | - | - | NA | NA | NA |
| Methyl-αD-Mannopyranoside | API 50CH | - | NA | NA | NA | NA |
| Methyl-αD-Glucopyranoside | API 50CH | - | NA | NA | NA | NA |
| N-Acetylglucosamine | API 50CH | + | NA | NA | NA | d |
| Amygdalin | API 50CH | + | + | + | + | d |
| Esculin ferric citrate | API 50CH | + | + | NA | NA | d |
| Salicin | API 50CH | + | NA | + | + | d |
| D-Cellobiose | API 50CH | + | + | + | + | d |
| D-Maltose | API 50CH | + | NA | d | + | NA |
| D-Lactose | API 50CH | + | NA | d | d | + |
| D-Saccharose | API 50CH | + | + | + | + | NA |
| D-Trehalose | API 50CH | + | NA | d | d | NA |
| Inulin | API 50CH | - | NA | NA | NA | NA |
| D-Melezitose | API 50CH | - | - | NA | NA | NA |
| Amidon | API 50CH | - | NA | NA | NA | NA |
| Glycogen | API 50CH | - | NA | NA | NA | NA |
| Xylitol | API 50CH | - | NA | NA | NA | NA |
| Gentiobiose | API 50CH | + | NA | NA | NA | + |
| D-Turanose | API 50CH | - | NA | NA | NA | NA |
| D-Lyxose | API 50CH | - | NA | NA | NA | NA |
| D-Tagatose | API 50CH | - | NA | NA | NA | - |
| D-Fucose | API 50CH | - | NA | NA | NA | NA |
| L-Fucose | API 50CH | - | NA | NA | NA | NA |
| D-Arabitol | API 50CH | - | NA | NA | NA | NA |
| L-Arabitol | API 50CH | - | NA | NA | NA | NA |
| Potassium Gluconate | API 50CH | - | NA | NA | NA | NA |
| Potassium 2-ketogluconate | API 50CH | - | NA | NA | NA | NA |
| Potassium 5-ketogluconate | API 50CH | - | NA | NA | NA | NA |

In a further embodiment said strain or culture is susceptible to Gentamicin, Kanamycin, Streptomycin, Tetracycline, Erythromycin, Clindamycin, Chloramphenicol, Ampicillin and Vancomycin analysed according to ISO10932:2010 (when suitable) standard with VetMIC Lact-1 and VetMIC Lact-2 plates (SVA National Veterinary Institute, Uppsala, Sweden) in anaerobic conditions at +37°C for 48 hours.

In one embodiment the strain or the culture comprising the strain is characterized by 16 S RNA sequence defined by SEQ ID NO: 4 (*Lactobacillus rodentium* 11822_5, deposited to the VTT Culture Collection under the accession number VTT E-173547), the ability to adhere the mucosa of gastrointestinal tract and the ability to use the sugars as follows

**Table 4**

| Test/characteristics | Test | Lactic acid bacteria 11822_5 | Bergeys manual *Lactobacillus jensenii* | Bergeys manual *Lactobacillus gasserii* | Bergeys manual *Lactobacillus johnsonii* | Killer et al. 2014 Lactobacillus rodentium |
|---|---|---|---|---|---|---|
| Glycerol | API 50CH | - | NA | NA | NA | NA |
| Erythrotritol | API 50CH | - | NA | NA | NA | NA |
| D-Arabinose | API 50CH | - | - | NA | NA | NA |
| L-Arabinose | API 50CH | - | NA | NA | NA | NA |
| D-xylose | API 50CH | - | - | NA | NA | NA |
| L-xylose | API 50CH | - | NA | NA | NA | NA |
| D-Adonitol | API 50CH | - | NA | NA | NA | NA |
| Methyl-βD-Xylanopyroside | API 50CH | - | NA | NA | NA | NA |
| D-Galactose | API 50CH | - | NA | + | + | d |
| D-Glucose | API 50CH | + | NA | NA | NA | NA |
| D-Fructose | API 50CH | + | NA | NA | NA | NA |
| D-Mannose | API 50CH | + | NA | + | + | d |
| L-Sorbose | API 50CH | - | NA | NA | NA | NA |
| L-Rhamnose | API 50CH | - | NA | NA | NA | NA |
| Dulcitol | API 50CH | - | NA | NA | NA | NA |
| Inositol | API 50CH | - | NA | NA | NA | NA |
| D-Mannitol | API 50CH | - | d | - | - | NA |
| D-Sorbitol | API 50CH | - | - | NA | NA | NA |
| Methyl-αD-Mannopyranoside | API 50CH | - | NA | NA | NA | NA |
| Methyl-αD-Glucopyranoside | API 50CH | - | NA | NA | NA | NA |
| N-Acetylglucosamine | API 50CH | + | NA | NA | NA | d |
| Amygdalin | API 50CH | + | + | + | + | d |
| Arbutin | API 50CH | + | NA | NA | NA | - |
| Esculin ferric citrate | API 50CH | + | + | NA | NA | d |
| Salicin | API 50CH | + | NA | + | + | d |
| D-Cellobiose | API 50CH | + | + | + | + | d |
| D-Maltose | API 50CH | + | NA | d | + | NA |
| D-Lactose | API 50CH | + | NA | d | d | + |
| D-Melibiose | API 50CH | - | - | d | d | + |
| D-Saccharose | API 50CH | + | + | + | + | NA |
| D-Trehalose | API 50CH | + | NA | d | d | NA |
| Inulin | API 50CH | - | NA | NA | NA | NA |
| D-Melezitose | API 50CH | - | - | NA | NA | NA |
| D-Raffinose | API 50CH | - | - | d | d | + |
| Amidon | API 50CH | - | NA | NA | NA | NA |
| Glycogen | API 50CH | - | NA | NA | NA | NA |
| Xylitol | API 50CH | - | NA | NA | NA | NA |
| Gentiobiose | API 50CH | + | NA | NA | NA | + |
| D-Turanose | API 50CH | - | NA | NA | NA | NA |
| D-Lyxose | API 50CH | - | NA | NA | NA | NA |
| D-Tagatose | API 50CH | - | NA | NA | NA | - |
| D-Fucose | API 50CH | - | NA | NA | NA | NA |
| L-Fucose | API 50CH | - | NA | NA | NA | NA |
| D-Arabitol | API 50CH | - | NA | NA | NA | NA |
| L-Arabitol | API 50CH | - | NA | NA | NA | NA |
| Potassium Gluconate | API 50CH | - | NA | NA | NA | NA |
| Potassium 2-ketogluconate | API 50CH | - | NA | NA | NA | NA |
| Potassium 5-ketogluconate | API 50CH | - | NA | NA | NA | NA |

In a further embodiment said strain or culture is susceptible to Gentamicin, Kanamycin, Streptomycin, Tetracycline, Erythromycin, Clindamycin, Chloramphenicol, Ampicillin and Vancomycin analysed according to ISO10932:2010 (when suitable) standard with VetMIC Lact-1 and VetMIC Lact-2 plates (SVA National Veterinary Institute, Uppsala, Sweden) in anaerobic conditions at +37°C for 48 hours.

In one embodiment the strain or the culture comprising the strain is characterized by 16 S RNA sequence defined by SEQ ID NO: 5 (*Lactobacillus rodentium* 11822_8, deposited to the VTT Culture Collection under the accession number VTT E-173548), the ability to adhere the mucosa of gastrointestinal tract and the ability to use the sugars as follows

**Table 5**

| Test/characteristics | Test | Lactic acid bacteria 11822_8 | Bergeys manual *Lactobacillus jensenii* | Bergeys manual *Lactobacillus gasserii* | Bergeys manual *Lactobacillus johnsonii* | Killer et al. 2014 Lactobacillus rodentium |
|---|---|---|---|---|---|---|
| Glycerol | API 50CH | - | NA | NA | NA | NA |
| Erythrotritol | API 50CH | - | NA | NA | NA | NA |
| D-Arabinose | API 50CH | - | - | NA | NA | NA |
| L-Arabinose | API 50CH | + | NA | NA | NA | NA |
| D-ribose | API 50CH | + | + | NA | NA | NA |
| D-xylose | API 50CH | + | - | NA | NA | NA |
| L-xylose | API 50CH | - | NA | NA | NA | NA |
| D-Adonitol | API 50CH | - | NA | NA | NA | NA |
| Methyl-βD-Xylanopyroside | API 50CH | - | NA | NA | NA | NA |
| D-Galactose | API 50CH | + | NA | + | + | d |
| D-Glucose | API 50CH | + | NA | NA | NA | NA |
| D-Fructose | API 50CH | + | NA | NA | NA | NA |
| D-Mannose | API 50CH | + | NA | + | + | d |
| L-Sorbose | API 50CH | - | NA | NA | NA | NA |
| Dulcitol | API 50CH | - | NA | NA | NA | NA |
| Inositol | API 50CH | - | NA | NA | NA | NA |
| D-Sorbitol | API 50CH | - | - | NA | NA | NA |
| Methyl-αD-Mannopyranoside | API 50CH | + | NA | NA | NA | NA |
| N-Acetylglucosamine | API 50CH | + | NA | NA | NA | d |
| Amygdalin | API 50CH | + | + | + | + | d |
| Esculin ferric citrate | API 50CH | + | + | NA | NA | d |
| Salicin | API 50CH | + | NA | + | + | d |
| D-Cellobiose | API 50CH | + | + | + | + | d |
| D-Maltose | API 50CH | + | NA | d | + | NA |
| D-Lactose | API 50CH | + | NA | d | d | + |
| D-Saccharose | API 50CH | + | + | + | + | NA |
| D-Trehalose | API 50CH | + | NA | d | d | NA |
| Inulin | API 50CH | - | NA | NA | NA | NA |
| D-Melezitose | API 50CH | - | - | NA | NA | NA |
| Glycogen | API 50CH | - | NA | NA | NA | NA |
| Xylitol | API 50CH | - | NA | NA | NA | NA |
| Gentiobiose | API 50CH | + | NA | NA | NA | + |
| D-Turanose | API 50CH | - | NA | NA | NA | NA |
| D-Lyxose | API 50CH | - | NA | NA | NA | NA |
| D-Tagatose | API 50CH | - | NA | NA | NA | - |
| D-Fucose | API 50CH | - | NA | NA | NA | NA |
| L-Fucose | API 50CH | - | NA | NA | NA | NA |
| D-Arabitol | API 50CH | - | NA | NA | NA | NA |
| L-Arabitol | API 50CH | - | NA | NA | NA | NA |
| Potassium Gluconate | API 50CH | + | NA | NA | NA | NA |
| Potassium 2-ketogluconate | API 50CH | - | NA | NA | NA | NA |
| Potassium 5-ketogluconate | API 50CH | - | NA | NA | NA | NA |

In a further embodiment said strain or culture is susceptible to Gentamicin, Kanamycin, Streptomycin, Tetracycline, Erythromycin, Clindamycin, Chloramphenicol, Ampicillin and Vancomycin analysed according to ISO10932:2010 (when suitable) standard with VetMIC Lact-1 and VetMIC Lact-2 plates (SVA National Veterinary Institute, Uppsala, Sweden) in anaerobic conditions at +37°C for 48 hours.

One embodiment provides said strain or said culture, wherein the strain is susceptible to one or more of the antibiotics in amount listed below in Table 6.

**Table 6**

| Strain | *Lactococcus lactis ssp.cremoris* 11809_6 | EFSA MIC cut-off µg/ml | *Lactobacillus rodentium* 11822_3 | *Lactobacillus rodentium* 11822_4 | *Lactobacillus rodentium* 11822_5 | *Lactobacillus rodentium* 11822_8 | EFSA MIC cut-off µg/ml |
|---|---|---|---|---|---|---|---|
| SEQ ID NO: | 1 | | 2 | 3 | 4 | 5 | |
| Gentamicin | 1 | 32 | 0.5 | 0.5 | 0.5 | 0.5 | 16 |
| Kanamycin | 8 | 64 | 16 | 16 | 32 | 16 | 64 |
| Streptomycin | 32 | 32 | 4 | 4 | 4 | 4 | 16 |
| Tetracycline | 1 | 4 | 8 | 8 | 8 | 8 | 4 |
| Erythromycin | 0.12 | 1 | 0.06 | 0.06 | 0.12 | 0.06 | 1 |
| Clindamycin | 0.06 | 1 | 0.06 | 0.06 | 0.06 | 0.06 | 1 |
| Chloramphenicol | 4 | 8 | 4 | 4 | 4 | 4 | 4 |
| Ampicillin | 0.25 | 2 | 0.5 | 1 | 0.5 | 1 | 1 |
| Vancomycin | 0.5 | 4 | 1 | 1 | 1 | 1 | 2 |

| | | | | | | | |
|---|---|---|---|---|---|---|---|
| **n.r. not relevant** | | | | | | | |

Preferably the culture is essentially pure from other lactic acid bacteria and any other microbes or impurities. A pure culture can be obtained using conventional microbiological methods. However, the final preparation may contain any other auxiliary agents depending on the formulation, such as residues of the culture media, carriers, stabilizers, preservatives or e.g. cryoprotectants.

In one embodiment the lactic acid bacterial strain is *Lactococcus lactis,* such as *Lactococcus lactis ssp. cremoris.* Specifically *Lactococcus lactis ssp. cremoris* 11809_6 is defined by having a sequence having SEQ ID NO: 1.

In one embodiment the lactic acid bacterial strain is *Lactobacillus rodentium* 11822_3 is defined by having a sequence having SEQ ID NO: 2; *Lactobacillus rodentium* 11822_4 is defined by having a sequence having SEQ ID NO: 3; *Lactobacillus rodentium* 11822_5 is defined by having a sequence having SEQ ID NO: 4; and *Lactobacillus rodentium* 11822_8 is defined by having a sequence having SEQ ID NO: 5.

In order to be usable as a probiotic the strain must meet also other criteria in addition to viability and ability to adhere. The strain should not be resistant to antibiotics; susceptibility profiles of the strains here described are discussed in the experimental section but exact Minimal Inhibitory Concentration (MIC)-values may vary.

The strain must tolerate the acidic pH of stomach without losing viability. According to one embodiment the strain maintains its viability at pH 3 for 2 hours. In this connection maintained viability means that a viable count after subjection to acid conditions is at least 60%, preferably at least 70%, more preferably at least 80% and most preferably 90% from the viable count of strains which have not been subjected to acid conditions.

The strain must tolerate certain amount of bile acids without losing viability. According to one embodiment the strain maintains its viability at 0.3 *wt*-% and 1.0 *wt*-% bile concentrations; more specifically the growth of the strains in bile acid concentration of 0.3 *wt*-% is at least 20%, preferably at least 30%, more preferably at least 40% of the growth.

One embodiment provides a food (feed) composition or a nutritional supplement, also called as dietary supplement, for example for canine, which comprises one or more of said strain(s) or said culture(s). The food composition or the nutritional supplement may be also called as a food product. A nutritional or dietary supplement is intended to provide one or more nutrients, prebiotic or probiotics that may otherwise not be consumed in sufficient quantities. The food composition or nutritional supplement may contain as auxiliary agents, fillers, amino acids and/or proteins, fatty acids, carbohydrates, vitamins, minerals, food products, pharmaceutical agents, preservatives, flavours, aroma or the like. Food/feed compositions include canine foods in any form. Examples of nutritional supplement include tablets, chewing products, snacks, treats, liquids, such as fermented milk based products, for example sour milk, yoghurt, kefir, sour whole milk, curd and the like milk products. Fermented milk products, which also known as cultured dairy foods, cultured dairy products, or cultured milk products, are dairy food products that have been fermented with lactic acid bacteria such as *Lactobacillus, Lactococcus,* and *Leuconostoc.*

In one embodiment the composition is a milk product, such as fermented milk product or other dairy product. Fermented milk products naturally contain lactic acid providing good storage properties and various other aromatic substances.

In one embodiment the composition or supplement contains also other probiotic strains including various lactic acid bacteria, Bifidobacteria, and/or Saccharomyces Especially when the strain or culture described here is used in fermented dairy products it should be compatible with fermenting microbes and possible further probiotic strains.

One embodiment provides the composition or the supplement, such as the milk product, wherein said composition or supplement further comprises a starter culture. The starter culture may be any known starter culture, such as a culture containing thermophilic and/or mesophilic microbes. The cultures may be of single-strain type containing only one strain of bacteria, or multiple-strain type which is a mixture of several strains, each with its own specific effect. In the present study the strains have been shown to be usable with various commercial starter cultures, i.e. they do not compromise growth and fermentative activity of the starter culture. They also maintain their own viability and ability to adhere.

One embodiment is a method for preparation of the milk based composition described here. Said method comprising the steps of:
a) fat standardization; and
b) protein-standardization; and
c) homogenization; and
d) pasteurization; and
e) cooling; and
f) inoculation with starter cultures; and
g) incubation and cooling; and
h) stirring; and
i) pasteurization; and
j) inoculation with the strain or a culture described here; and
k) packing.

Non-limiting examples of preparation of various fermented dairy product are given below.

### Sourmilk (mesophilic) / stirred

- Fat standardization (0.05 - 5.00 wt-%)
- Protein-standardization (3.50 - 10.00 *wt-*%)
- Homogenization
- Pasteurization (72 °C / 15 sec. - 92 °C / 15 min.)
- Cooling to 20 - 30 °C
- Inoculation with mesofilic starter cultures and probiotic(s)
- Incubation at 20 - 30 °C / 8 - 22 h
- Cooling when pH 4.6 - 4.4 (SH 36 - 38)
- Stirring
- Packing.

### Sourmilk (thermophilic) / stirred

- Fat standardization (0.05 - 10.00 *wt*-%)
- Protein-standardization (3.50 - 10.00 *wt*-%)
- Homogenization
- Pasteurization (72 °C / 15 sec. - 92 °C / 15 min.)
- Cooling to 32 - 44 C
- Inoculation with thermofilic starter cultures and probiotic(s)
- Incubation at 32 - 44 °C / 4 - 12 h
- Cooling when pH 4.8 - 4.4 (SH 36 - 38)
- Stirring
- Packing

### Sourmilk (mesophilic) / stirred / long life

- Fat standardization (0,05 - 5,00 *wt-*%)
- Protein-standardization (3.50 - 10.00 *wt-*%)
- Homogenization
- Pasteurization (72 °C / 15 sec. - 92 °C / 15 min.)
- Cooling to 20 - 30 °C
- Inoculation with mesofilic starter cultures
- Incubation at 20 - 30 °C / 8 - 22 h
- Cooling when pH 4.6 - 4.4 (SH 36 - 38)
- Stirring
- Pasteurization (72 °C / 15 sec. - 92 °C / 15 min.)
- Inoculation with probiotic(s) as a shot in packing line
- Packing

### Sourmilk (thermophilic) / stirred / long life

- Fat standardization (0,05 - 10,00 *wt-*%)
- Protein-standardization (3,50 - 10,00 *wt-*%)
- Homogenization
- Pasteurization (72 °C / 15 sec. - 92 °C / 15 min.)
- Cooling to 32 - 44 C
- Inoculation with thermofilic starter cultures
- Incubation at 32 - 44 °C / 4 - 12 h
- Cooling when pH 4.8 - 4.4 (SH 36 - 38)
- Stirring
- Pasteurization (72 °C / 15 sec. - 92 °C / 15 min.)
- Inoculation with probiotic(s) as a aseptic-shot in packing line
- packing.

Suitable strains for a starter culture are for example *Lactococcus lactis* ssp. *cremoris, Lactococcus lactis* spp. *lactis* and *Leuconostoc* ssp., alone or in a combination. In on eembodiment the starter culture consists of the above listed strains.

In one embodiment the composition is a feed or food for canine, in general dog food, such as dry feed, fresh or wet feed, or a supplement. The terms "feed" and "food" may be used interchangeably. The dry form of the feed may be called for example as kibble. Wet form of the feed may be provided for example as canned form, which is significantly higher in moisture than dry or semi-moist food.

One embodiment provides the composition or the supplement, wherein the bacteria are lyophilized or spray dried.

Basic steps of production of a composition is dry form typically include standardization of fat and protein, milling, drying in e.g. spray towers, cooling to 10 - 40 °C and inoculation with probiotics as a aseptic-shot in packing line. In order to maintain their biological activity the strains, lyophilized, dried, or liquid culture, can be introduced aseptically to heat-treated food/feed composition. One embodiment provides the composition or the supplement, wherein the bacteria are lyophilized or spray dried.

The strains disclosed herein may be used in methods wherein the strain, or a culture containing said strain, is to be administered to a subject in need thereof. The strain or the culture may be in the form of any of the food products described herein.

One embodiment provides a method for maintaining or improving microbial flora in gastrointestinal tract comprising administering an effective amount of said strain or said culture to a subject, such as to a canine.

The effective amount as used herein refers to a daily dose which is sufficient for providing and maintaining a positive effect in the subject after either a single treatment or multiple administrations.

One embodiment provides a method for maintaining or improving health and/or wellbeing of immune system or gastrointestinal tract, comprising administering an effective amount of said strain or said culture to a subject, such as to a canine. One embodiment provides a method for treating or preventing diarrhea and food sensitivities comprising of the administration of an effective amount of said strain or said culture to a subject, such as a canine.

In one embodiment in the method the effective amount is 1x10⁷ cfu per serving or per day; preferably 2x10⁸ cfu per day. In one embodiment the effective amount is 1,5x10⁹ cfu per day.

One embodiment provides use of lactic acid bacteria characterized by 16 S RNA sequence defined by SEQ ID NO: 1, SEQ ID NO: 2, SEQ ID NO: 3, SEQ ID NO: 4 or SEQ ID NO: 5 for maintaining intestinal health, or for treatment and/or prophylactics of diarrhea, or for any other purpose as described herein.

One embodiment provides use of lactic acid bacteria characterized by 16 S RNA sequence defined by SEQ ID NO: 1, SEQ ID NO: 2, SEQ ID NO: 3, SEQ ID NO: 4 or SEQ ID NO: 5 for preparation of food composition or a nutritional supplement for canine, as described herein.

It is to be understood that the terminology employed herein is for the purpose of description and should not be regarded as limiting. It must be understood, that the embodiments given in the description above are for illustrative purposes only, and that various changes and modifications are possible within the scope of the disclosure.

The features described here as separate embodiments may also be provided in combination in a single embodiment. Also various features of the described here in the context of the method are usable in connection with the compositions and uses, and vice versa.

### Examples

### Example 1: Origin of samples and preliminary data

### Origin of the samples

Samples were collected from colonic samples (owned by Biosafe - Biological Safety Solutions Itd, Kuopio, Finland) from Finnish wolves (*Canis lupus*). In the beginning of the study there were samples from 20 wolves (13 males, 7 females), from which the colonic bacteria were isolated for identification and research for biological activities. More than 160 sample isolates were tested to find the most potential probiotic bacteria strains.

### Preliminary screening tests

The preliminary tests were physiological screening tests like gram-staining and enzyme (catalase, oxidase) activity measurement and further tests for the microorganisms identification to the species level (API test), growth and fermentation test in milk and antibiotic resistance characterization (results not shown here).

### Selected samples

In the table 7 below according to all tests done only the most potential probiotic bacteria for feed additives, and their origin, are listed.

**Table 7. List of the bacterial samples and the origin thereof. In the name of the sample the identified bacteria is mentioned and the numbers (5 number code) are the code of the wolf and the single number is the code of isolated bacteria colony meaning different species or strain.**

| **Sample (Identified bacteria)** | **Hunting date of the wolf** | **Place** | **Sex** | **Weight** |
|---|---|---|---|---|
| *Lactococcus lactis ssp. cremoris* 11809_6 | 24.2.2015 | Ilomantsi | female | 30.4 kg |
| *Lactobacillus rodentium* 11822_3 | 24.2.2015 | Kuhmo | female | 30 kg |
| *Lactobacillus rodentium* 11822_4 | 24.2.2015 | Kuhmo | female | 30 kg |
| *Lactobacillus rodentium* 11822_5 | 24.2.2015 | Kuhmo | female | 30 kg |
| *Lactobacillus rodentium* 11822_8 | 24.2.2015 | Kuhmo | female | 30 kg |

The sample 11809_6 contained the 16 S RNA sequence defined by SEQ ID NO: 1. The sample 11822_3 contained the 16 S RNA sequence defined by SEQ ID NO: 2. The sample 11822_4 contained the 16 S RNA sequence defined by SEQ ID NO: 3. The sample 11822_5 contained the 16 S RNA sequence defined by SEQ ID NO: 4. The sample 11822_8 contained the 16 S RNA sequence defined by SEQ ID NO: 5.

In the following test results, only the conclusions of these selected samples are shown.

### Example 2: Characterization of antibiotic susceptibility of unidentified lactic acid bacteria strains

Antibiotic susceptibility of the unidentified lactic acid bacteria strains were analysed according to ISO10932:2010 (when suitable) standard with VetMIC Lact-1 and VetMIC Lact-2 plates (SVA National Veterinary Institute, Uppsala, Sweden) in anaerobic conditions at +37 °C for 48 hours. Results of antibiotic susceptibility of strains are presented in table 8. While the strains have not been identified, the range of MIC cut-off values for *Lactobacillus* species and *Lactococcus lactis* required by EFSA (EFSA 2012. Guidance on the assessment of bacterial susceptibility to antimicrobials of human and veterinary importance. EFSA Journal 2012, 10(6), 2740) are presented as a reference value.

The MIC-values of unidentified lactic acid bacteria 11822_3, 11822_4, 11822_5 and 11822_8 were two times higher than MIC cutoff value for kanamycin (11822_3, 11822_4, 11822_5) and/or tetracycline (11822_5 and 11822_8) if the strains were to be identified as obligate homofermentative lactobacillus (in case of kanamycin and tetracycline resistance), a member of *Lactobacillus acidophilus* group (tetracycline), *Lactobacillus case*/*paracasei* (tetracycline) or *Lactococcus lactis.*

Regardless of the identification, the MIC values of unidentified lactic acid bacteria strain 11809_6 did not exceed MIC cut-off values.

**Table 8. Antibiotic susceptibility profile of unidentified lactic acid bacteria and the range of Minimal inhibitory Concentration (MIC)-values reported by European Food Safety Authority (EFSA) for Lactobacillus species and Lactococcus lactis. The MIC values marked with black color fulfills the EFSA requirements (max 2X MIC cutoff value). The MIC values marked with blue color may fulfill the EFSA requirement, depending on the species identification. The MIC values marked with the red color do not fulfill the EFSA requirements without explaining the molecular background of the resistance.**

| | 11809_6 | 11822_3 | 11822_4 | 11822_5 | 11822_8 | EFSA MIC range |
|---|---|---|---|---|---|---|
| Gentamicin | 1 | 0.5 | 0.5 | 1 | 0.5 | 8-32 |
| Kanamycin | 8 | 32 | 32 | 32 | 16 | 16-64 |
| Streptomycin | 16 | 4 | 4 | 4 | 4 | n.r., 16-64 |
| Tetracycline | 0.5 | 4 | 4 | 8 | 8 | 4-32 |
| Erythromycin | 0.12 | 0.12 | 0.12 | 0.03 | 0.03 | 1 |
| Clindamycin | 0.06 | 0.03 | 0.03 | 0.03 | 0.03 | 1-2 |
| Chloramphenicol | 4 | 2 | 2 | 2 | 2 | 4-8 |
| Ampicillin | 0.25 | 0.5 | 0.5 | 0.5 | 0.5 | 1-4 |
| Vancomycin | 0.5 | 1 | 1 | 1 | 1 | n.r., 2-4 |

| | | | | | | |
|---|---|---|---|---|---|---|
| **n.r. not relevant** | | | | | | |

### Conclusions

The MIC values of unidentified strains 11822_3, 11822_4, 11822_5 and 11822_8 exceeded EFSA MIC cutoff values, but while the values were only 2x cutoff value, the result is inside of method inaccuracy and accepted by EFSA.

Regardless of the identification, the MIC values of unidentified lactic acid bacteria strain 11809_6 did not exceed MIC cut-off values.

### References/ Characterization of antibiotic susceptibility

EFSA 2012. Guidance on the assessment of bacterial susceptibility to antimicrobials of human and veterinary importance. EFSA Jourmal 2012, 10(6), 2740.

### Identification of lactic acid bacteria

The objective of the study was to identify selected lactic acid bacteria via morphological, biochemical and molecular biology methods.

### Example 3: Identification of lactic acid bacteria 11809_6

### Methods and results

### Gram-staining

Lactic acid bacteria 11809_6 was inoculated on MRS agar and incubated anaerobically at +37 °C for 48 hours. Samples were transferred into fresh MRS agar plates and MRS broth and incubated at +37 °C for 24 hours. A bacterial colony was collected with a sterile loop from the MRS agar, smeared on the object glass and Gram stained.

Lactic acid bacteria 11809_6 is a Gram positive cocci/short rod shaped bacterium

### Oxidase and catalase tests

A bacterial colony was collected with a sterile loop from the MRS agar, smeared on the filter paper and 2 drops of oxidase reagents A and B (Oxidase reagent according to Gaby-Hadley A, lot BCBM3527V, Fluka; Oxidase reagent according to Gaby-Hadley B, lot BCBN3836V, Fluka) were added. Colour reaction was visually recorded for two minutes.

A bacterial colony was collected with a sterile loop from the MRS agar, smeared on the object glass and one drop 3% hydrogen peroxide was added on top.

Lactic acid bacteria 11809_6 is an oxidase negative and catalase negative bacterium.

### Use of sugars; Biochemical identification with API 50CH test strips

The biochemical identification for lactic acid bacteria 11809_6 and was performed with API 50 CH (Biomerieux, France) according to API 50 CHL medium (Biomerieux, France) instructions. In each case a discrete bacterial colony from a MRS plate was suspended into API suspension medium (2 ml ampoule).

This suspension was added drop by drop into API suspension medium (5 ml ampoule) until a suspension comparable to 2 MacFarland was achieved. Twice as many drops from this suspension was then added into API 50 CHL medium and API 50 CH strips were filled with this suspension and covered with mineral oil. The API CH strips were incubated at +37°C for 72 hours. The results were analysed in APIWEB web-software (Biomerieux, France).

**Table 9. Results of biochemical characterization tests of lactic acid bacteria 11809_6 and Bergey's manual (De Vos P, Garrity GM, Jones D, Krieg NR, Ludwig W, Rainey FA, Schleifer K-H & Whitman WB, 2009. Bergey's manual of systematic bacteriology, 2nd edition, volume 3 The Firmicutes. Springer, Athens, USA, 1450 pages) biochemical characterization data of three closest matching species (Lactococcus lactis ssp lactis, Lactococcus lactis ssp. cremoris) in 16S rRNA gene sequence data comparison. The divergent results from lactic acid bacteria 11809_6 are marked using parenthesis.**

| Test/characteristics | Test | Lactic acid bacteria 11809_6 | Bergeys manual | Bergeys manual |
|---|---|---|---|---|
| | | | *Lactococcus lactis* ssp. *cremoris* | *Lactococcus lactis* ssp. *lactis* |
| Glycerol | API 50CH | - | NA | NA |
| Erythrotritol | API 50CH | - | NA | NA |
| D-Arabinose | API 50CH | - | NA | NA |
| L-Arabinose | API 50CH | - | NA | NA |
| D-ribose | API 50CH | + | (-) | + |
| D-xylose | API 50CH | + | NA | NA |
| L-xylose | API 50CH | - | NA | NA |
| D-Adonitol | API 50CH | - | NA | NA |
| Methyl-βD-Xylanopyroside | API 50CH | - | NA | NA |
| D-Galactose | API 50CH | +/- | + | + |
| D-Glucose | API 50CH | + | NA | NA |
| D-Fructose | API 50CH | + | NA | NA |
| D-Mannose | API 50CH | + | NA | NA |
| L-Sorbose | API 50CH | - | NA | NA |
| L-Rhamnose | API 50CH | - | NA | NA |
| Dulcitol | API 50CH | - | NA | NA |
| Inositol | API 50CH | - | NA | NA |
| D-Mannitol | API 50CH | + | NA | NA |
| D-Sorbitol | API 50CH | - | NA | NA |
| Methyl-αD-Man nopyranoside | API 50CH | - | NA | NA |
| Methyl-αD-Glucopyranoside | API 50CH | - | NA | NA |
| N-Acetylglucosamine | API 50CH | + | NA | NA |
| Amygdalin | API 50CH | +/- | NA | NA |
| Arbutin | API 50CH | + | NA | NA |
| Esculin ferric citrate | API 50CH | + | NA | NA |
| Salicin | API 50CH | + | NA | NA |
| D-Cellobiose | API 50CH | + | NA | NA |
| D-Maltose | API 50CH | + | (-) | + |
| D-Lactose | API 50CH | + | + | + |
| D-Melibiose | API 50CH | - | - | - |
| D-Saccharose | API 50CH | - | NA | NA |
| D-Trehalose | API 50CH | + | NA | NA |
| Inulin | API 50CH | - | NA | NA |
| D-Melezitose | API 50CH | - | - | - |
| D-Raffinose | API 50CH | - | - | - |
| Amidon | API 50CH | +/- | NA | NA |
| Glycogen | API 50CH | - | NA | NA |
| Xylitol | API 50CH | - | NA | NA |
| Gentiobiose | API 50CH | + | NA | NA |
| D-Turanose | API 50CH | - | NA | NA |
| D-Lyxose | API 50CH | - | NA | NA |
| D-Tagatose | API 50CH | - | NA | NA |
| D-Fucose | API 50CH | - | NA | NA |
| L-Fucose | API 50CH | - | NA | NA |
| D-Arabitol | API 50CH | - | NA | NA |
| L-Arabitol | API 50CH | - | NA | NA |
| Potassium Gluconate | API 50CH | +/- | NA | NA |
| Potassium 2-ketogluconate | API 50CH | - | NA | NA |
| Potassium 5-ketogluconate | API 50CH | - | NA | NA |

In Bergey's manual data: + 90% or more strains are positive, - 90% or more strains are negative, d 11-89% of strains are positive, NA data not available in Bergey's manual. The model color reactions for both positive and negative reactions are described by manufacturer. In case the color reaction does not match with either negative or positive reaction, the test value is marked with +/-.

According to APIWEB the closest match for lactic acid bacteria 11809_6 was *Lactococcus lactis* ssp. *lactis*

### Genomic DNA isolation

Genomic DNA was isolated from lactic acid bacteria 11809_6 with the Gene JET genomic DNA purification kit (Thermo Scientific, Vilnius, Lithuania, Lot 00248461) according to manufacturer's instructions (method E:Gram-positive bacteria) from MRS-medium incubated at +37°C for 24 hours.

### 16S rRNA gene sequencing

Strain identification was performed by PCR amplification of 16S rRNA gene with universal primers 27f (5' AGA GTT TGA TCC TGG CTC AG 3; SEQ ID NO: 6') and 1492r (5' ACG GCT ACC TTG TTA CGA CTT 3'; SEQ ID NO: 7) (Weisburg, WG, Barns, SM, Pelletier, DA. & Lane, DJ, 1991. 16S ribosomal DNA amplification for phylogenetic study. Journal of Bacteriology 173: 697-7). The amplification reactions were performed with a slightly modified method of Korkea-aho et al. (Korkea-aho, TL, Heikkinen, J, Thompson, KD, von Wright, A & Austin, B, 2011. Pseudomonas sp. M174 inhibits the fish pathogen Flavobacterium psychrophilum. Journal of Applied Microbiology, 111, 266-277), in volumes of 50 µl containing 36.5 µl Direct-Q water (Direct-Q 5UV-R, Merck-Millipore, Darmstadt, Germany), 5 µl of 10X PCR buffer (Thermo Scientific, Vilnius Lithuania), 3 µl 25 mM MgCl₂ (Thermo Scientific, Vilnius Lithuania), 1 µl 10mM dNTP mix (Thermo Scientific, Vilnius, Lithuania), 1.25 µl of each 10 µM primer (Oligomer, Helsinki, Finland), 2 units of Dynazyme II polymerase (Thermo Scientific, Vilnius Lithuania) and 1 µl of template.

PCR amplification was carried out in 0.2 ml nuclease-free PCR tubes using Techne FtcPlus/02 thermal cycler (Bibby Scientific, UK) programmed as follows: initial denaturing step at 94°C 2 minutes, followed by 40 cycles of 94°C for 40 seconds, annealing at 56°C for 90 seconds and extension at 72°C for 1 minute. The final extension was at 72°C for 10 minutes. The correct size of the PCR product was confirmed with 0.9% agarose gel electrophoresis and PCR product was cleaned with GeneJET Gel Extraction Kit (Thermo Scientific, Vilnius Lithuania). Purified PCR product was sequenced in LGC Genomics (Berlin, Germany). The sequences were analyzed with Geneious 8.1.6 (Biomatters, New Zealand), poor quality sequence was removed from both ends and compared to the NCBI reference genomic database and Nucleotide collection database using Basic Local Alignment Search Tool (BLAST) (http://blast.ncbi.nlm.nih.gov/Blast.cgi).

NCBI's Reference Sequence (RefSeq) database is a collection of taxonomically diverse, non-redundant and richly annotated sequences representing naturally occurring molecules of DNA, RNA, and protein. Included are sequences from plasmids, organelles, viruses, archaea, bacteria, and eukaryotes. Each RefSeq is constructed wholly from sequence data submitted to the International Nucleotide Sequence Database Collaboration (INSDC). Similar to a review article, a RefSeq is a synthesis of information integrated across multiple sources at a given time. RefSeqs provide a foundation for uniting sequence data with genetic and functional information. They are generated to provide reference standards for multiple purposes ranging from genome annotation to reporting locations of sequence variation in medical records. NCBI Genomic Reference Sequence database contains 17,841,085 sequences (http://www.ncbi.nlm.nih.gov/books/NBK21091/, 15.3.2016).

NCBI's Nucleotide collection (nr/nt) database is the largest available non-redundant BLAST database. NCBI Nucleotide collection database contains 35,150,479 sequences (http://www.ncbi.nlm.nih.gov/books/NBK21091/, 15.3.2016).
Lactic acid bacteria 11809_6, 27f sequence, 940bp; is shown as SEQ ID NO: 1

The comparison of the sequences with NCBI reference genomic database provided 100% match with five *Lactococcus lactis* ssp. *cremoris* sequence.

The comparison of the sequences with NCBI nucleotide collection database provided 100% match with fifteen *Lactococcus lactis* ssp. *cremoris* sequences and two *Lactococcus lactis* ssp. *lactis* sequence

### Conclusions

16S rRNA gene sequence showed 100 % match with *Lactococcus lactis* ssp. *cremoris.* However, the biochemical characterization with API 50CHL indicated that the strain 11809_6 would be *Lactococcus lactis* ssp. *lactis,* because the strain showed a capability to utilize ribose and maltose.

### Summary of the results of the identification of the samples:

### Results of identification of lactic acid bacteria 11809_6

*Gram-staining:* Lactic acid bacterium 11809_6 is a Gram positive cocci/short rod shaped bacterium

*Oxidase and catalase tests:* Lactic acid bacteria 11809_6 is an oxidase negative and catalase negative bacterium.

*Biochemical identification with API 50CH test strips:* According to APIWEB the closest match for lactic acid bacteria 11809_6 was *Lactococcus lactis* ssp. *lactis. 16S rRNA gene sequencing:* The comparison of the sequences with NCBI reference genomic database provided 100% match with five *Lactococcus lactis* ssp. *cremoris* sequence.

The comparison of the sequences with NCBI nucleotide collection database provided 100% match with fifteen *Lactococcus lactis* ssp. *cremoris* sequences and two *Lactococcus lactis* ssp. *lactis* sequence

### Conclusions for 11809_6

16S rRNA gene sequence showed 100 % match with *Lactococcus lactis* ssp. *cremoris.* However, the biochemical characterization with API 50CHL indicated that the strain 11809_6 would be *Lactococcus lactis* ssp. *lactis,* because the srain showed capability to utilize ribose and maltose.

### Example 4: Identification of strains 11822_3, 11822_4, 11822_5 and 11822_8

Samples were identified as described in Example 3. Results for each strain are summarized below.

### Results of identification of lactic acid bacteria 11822_3

*Gram-staining:* Lactic acid bacterium 11822_3 is a Gram positive long, rod shaped bacterium

*Oxidase and catalase tests:* Lactic acid bacterium 11822_3 is an oxidase negative and catalase negative bacterium.

*Biochemical identification with API 50CH test strips:* According to APIWEB the closest match for lactic acid bacteria 11822_3 was *Lactobacillus delbrueckii* ssp. *lactis,* see Figure 1.

*16S rRNA gene sequencing:* The comparison of the sequence (SEQ ID NO: 2) with NCBI reference genomic database provided 97.6% match with *Lactobacillus jensenii* sequence and 97.0-97.2% match with several *Lactobacillus johnsonii, Lactobacillus hominis* and *Lactobacillus gasserii* sequences. The comparison of the sequences with NCBI nucleotide collection database provided 100% match with one *Lactobacillus rodentium* sequence and 99.3 % match with two *Lactobacillus rodentium* sequences.

### Conclusions for 11822_3

16S rRNA gene sequence showed 100 % match with *Lactobacillus rodentium* sequence. The species was reported first time in 2014 and no biochemical profile is available in Bergey's manual yet.

### Results of identification of lactic acid bacteria 11822_4

*Gram-staining:* Lactic acid bacteriaum 11822_4 is a Gram positive long, rod shaped bacterium

*Oxidase and catalase tests:* Lactic acid bacteria 11822_4 is an oxidase negative and catalase negative bacterium.

*Biochemical identification with API 50CH test strips:* According to APIWEB the closest match for lactic acid bacteria 11822_4 was *Lactobacillus delbrueckii* ssp. *lactis;* see Figure 1.

*16S rRNA gene sequencing:* The comparison of the sequence (SEQ ID NO: 3) with NCBI reference genomic database provided 98.3% match with *Lactobacillus jensenii* sequence and 97.7-97.8% match with several *Lactobacillus johnsonii, Lactobacillus hominis* and *Lactobacillus gasserii* sequences.

The comparison of the sequences with NCBI reference genomic database provided 100% match with one *Lactobacillus rodentium* sequence and 99.5 % match with two *Lactobacillus rodentium* sequences.

### Conclusions for 11822_4

16S rRNA gene sequence showed 100% match with *Lactobacillus rodentium* sequence. The species is reported first time 2014 and no biochemical profile is available in Bergey's manual yet.

### Results of identification of lactic acid bacteria 11822_5

*Gram-staining:* Lactic acid bacterium 11822_5 is a Gram positive long, rod shaped bacterium

*Oxidase and catalase tests:* Lactic acid bacteria 11822_5 is an oxidase negative and catalase negative bacterium.

*Biochemical identification with API 50CH test strips:* According to APIWEB the closest match for lactic acid bacteria 11822_5 was *Lactobacillus delbrueckii* ssp. *lactis,* see Figure 1.

*16S rRNA gene sequencing:* The comparison of the sequence (SEQ ID NO: 4) with NCBI reference genomic database provided 98.3% match with *Lactobacillus jensenii* sequence and 97.7-97.8% match with several *Lactobacillus johnsonii, Lactobacillus hominis* and *Lactobacillus gasserii* sequences.

The comparison of the sequences with NCBI reference genomic database provided 100% match with one *Lactobacillus rodentium* sequence and 99.5 % match with two *Lactobacillus rodentium* sequences.

### Conclusions for 11822_5

16S rRNA gene sequence showed 100 % match with *Lactobacillus rodentium* sequence. The species is reported first time 2014 and no biochemical profile is available in Bergey's manual yet.

### Results of identification of lactic acid bacteria 11822_8

*Gram-staining:* Lactic acid bacterium 11822_8 is a Gram positive long, rod shaped bacterium

*Oxidase and catalase tests:* Lactic acid bacteria 11822_8 is an oxidase negative and catalase negative bacterium.

*Biochemical identification with API 50CH test strips:* According to APIWEB the closest match for lactic acid bacteria 11822_8 was *Lactococcus lactis* ssp. *lactis,* see Figure 1.

*16S rRNA gene sequencing:* The comparison of the sequence (SEQ ID NO: 5) with NCBI reference genomic database provided 98.3% match with *Lactobacillus jensenii* sequence and 98.1% match with two *Lactobacillus hominis* sequences. The comparison of the sequences with NCBI reference genomic database provided 100% match with one *Lactobacillus rodentium* sequence and 99.5 % match with two *Lactobacillus rodentium* sequences.

### Conclusions for 11822_8

16S rRNA gene sequence showed 100 % match with *Lactobacillus rodentium* sequence. The species is reported first time 2014 and no biochemical profile is available in Bergey's manual yet.

### Example 5: Antibacterial activity of lactic acid bacteria strains against Sacco Lyofast M442N, Y336A and AB1 souring agents in vitro

Objective of the study was to evaluate antimicrobial activity of lactic acid bacteria strains, 11809_6, 11822_3, 11822_4, 11822_5 and 11822_8 against souring agents Lyofast M442N, Y336A and AB1 *in vitro.*

### Methods and results

Lactic acid bacteria was inoculated on MRS agar and incubated anaerobically at +37 °C for 48 hours. Samples were transferred into MRS broth and incubated at +37 °C for 24 hours.

Starters Sacco Lyofast M442N and Y336 A were added into5 ml M17 broth + 0.5% glucose and Starter AB1 into MRS broth and incubated at +37 °C for 24 hours.

The antimicrobial porperties of the strains against the starters were detected on plate test in which the starter culture was streaked vertically with sterile loop (1µl) on the M17+0.5 % glucose (M442N and Y336A) or MRS plates (AB1) 5 lines per plate (Figure 1) as three replicates . At each plate, 5 different lactic acid bacteria strains were streaked horizontally with sterile loop (1 µl).

While the optimal groth temperature of the tested lactic acid bacteria was different compared to starters M442N (optimal temperature 25-32 °C) and Y336A (optimal temperature 43 °C), the test was performed in the temperatures described in table 10.

**Table 10. The test conditions of antimicrobial activity assay**

| The starter | Tested temperatures | Incubation time, h |
|---|---|---|
| M442N | +32 °C, +37 °C | 48 |
| Y336A | +37°C, +43°C | 48 |
| AB1 | +37°C | 48 |

Lactic acid bacteria strains 11809_6, 11822_3, 11822_4, 11822_5 and 11822_8 did not prevent the growth of the starters in any of the tested temperatures. Furthermore, no growth inhibition of the lactic acid bacteria strains by the starters was detected.

### Conclusions

No antimicrobial activity of lactic acid bacteria strains 11809_6, 11822_3, 11822_4, 11822_5 and 11822_8 was detectable against the starters Lyofast M442N, Y336A or AB1 at tested temperatures. Furthermore, the starters did not prevent the growth of any of the lactic acid bacteria strains.

### Example 6: Survival of lactic acid bacteria in 10% skim milk with and without Sacco Lyofast M442N, Y336A and AB1 starters in vitro

Objective of the study was to evaluate the survival of the lactic acid bacteria strains *Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822**_**8 in fermentation trial in 10% skim milk with and without starters Lyofast M442N, Y336A and AB1 *in vitro.*

### Methods and results

*Lactobacillus rodentium* 11822_3, *Lactobacillus rodentium* 11822_4, *Lactobacillus rodentium* 11822_5 and *Lactobacillus rodentium* 11822_8 were grown in MRS broth at +37 °C for 48 hours. Inoculation (1-2% depending on the turbidity of the culture) was made into fresh MRS broth and incubated at +37 °C for 24 hours. *Lactococcus lactis ssp. cremoris* 11809_6 was grown in M17+0.5% glucose broth for 48 hours and 1% inoculation was made into M17+0.5% glucose broth and culture was incubated at +37 °C for 24 hours.

Starters Sacco Lyofast M442N and Y336A were grown overnight in M17+0.5% glucose broth at +32 °C and +43 °C, respectively. Sacco Lyofast AB1 was grown overnight in MRS broth at +37°C.

The lactic acid bacteria and starter cultures were centrifuged 4000 rpm, 10 minutes, washed with sterile PBS and suspended into 1ml of sterile PBS. The test tubes for survival test in 5 ml of 10% skim milk fermentation were prepared according to Table 11.

**Table 11. The tested combinations in survival test and results of coagulation after overnight incubation and additional storage of the test tubes at +4 °C for 24 hours.**

| Added bacterium (200 µl) | Added starter (200 µl) | Added PBS | Milk | Incubation temperature | Coagulation after overnight incubation | Coagulation after additional 24h storage at +4 °C |
|---|---|---|---|---|---|---|
| *L. lactis* ssp *cremoris* 11809_6, 200µl | - | 200 µl | 5ml | +37°C | - | + |
| *L. rodentium* 11822-3, 200µl | - | 200 µl | 5ml | +37°C | - | + |
| *L. rodentium* 11822_4, 200µl | - | 200 µl | 5ml | +37°C | - | - |
| *L. rodentium* 11822_5, 200µl | - | 200 µl | 5ml | +37°C | - | - |
| *L. rodentium* 11822_8, 200µl | - | 200 µl | 5ml | +37°C | - | + |
| *L. lactis* ssp *cremoris* 11809_6, 200µl | M442N | - | 5ml | +32 °C | + | + |
| *L. lactis* ssp *cremoris* 11809_6, 200µl | Y336A | - | 5ml | +43 °C | + | + |
| *L. lactis* ssp *cremoris* 11809_6, 200µl | AB1 | - | 5ml | +37°C | + | + |
| *L. rodentium,* 11822_3, 200µl | M442N | - | 5ml | +32 °C | + | + |
| *L. rodentium* 11822_3, 200µl | Y336A | - | 5ml | +43 °C | + | + |
| *L. rodentium* 11822_3, 200µl | AB1 | - | 5ml | +37°C | + | + |
| *L. rodentium* 11822_4, 200µl | M442N | - | 5ml | +32 °C | + | + |
| *L. rodentium* 11822_4, 200µl | Y336A | - | 5ml | +43 °C | + | + |
| *L. rodentium* 11822_4, 200µl | AB1 | - | 5ml | +37°C | + | + |
| *L. rodentium* 11822_5, 200µl | M442N | - | 5ml | +32 °C | + | + |
| *L. rodentium* 11822_5, 200µl | Y336A | - | 5ml | +43 °C | + | + |
| *L. rodentium* 11822_5, 200µl | AB1 | - | 5ml | +37°C | + | + |
| *L. rodentium* 11822_8, 200µl | M442N | - | 5ml | +32 °C | + | + |
| *L. rodentium* 11822_8, 200µl | Y336A | - | 5ml | +43 °C | + | + |
| *L. rodentium* 11822_8, 200µl | AB1 | - | 5ml | +37C° | + | + |
| - | M442N | 200 µl | 5ml | +32 °C | + | + |
| - | Y336A | 200 µl | 5ml | +43 °C | + | + |
| - | AB1 | 200 µl | 5ml | +37C° | + | + |

All starter containing 10% skim milk tubes were coagulated after overnight incubation, while *Lactococcus lactis ssp cremoris* 11809_6, *Lactobacillus rodentium 11822_3, Lactobacillus rodentium* 11822_4, *Lactobacillus rodentium* 11822_5 and *Lactobacillus rodentium* 11822_8 did not separately coagulate the test milk. After additional 24 hour storage at +4 °C, the tubes containing *Lactococcus lactis ssp cremoris* 11809_6, *Lactobacillus rodentium* 11822_3 and *Lactobacillus rodentium* 11822_8 were coagulated.

The spread cultures was prepared from the tubes containing *Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822_8 separately and with each starter on MRS or M17+ 0.5% glucose agar and incubated for 48 hours. All bacteria survived the incubation in milk when they were incubated separately, but identification of test bacteria when they were incubated with starters was difficult, since the similar morphology of the test bacteria and the starter strains.

### Conclusions

*Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822_8 survived the incubation in milk. The tested strains have similar morphology with starter strains Sacco Lyofast M442N, Y336A and AB1, which prevents the detection after incubation of test strains and starters simultaneously.

### Example 7: pH tolerance assay of lactic acid bacteria strains in vitro (T207R228/2016, 31.3.2016)

Objective of the study was to evaluate the pH tolerance lactic acid bacteria strains, *Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822_8.

### Methods and results

*Enterococcus avium* 11805_4, *Enterococcus avium* 11805_6, *Lactobacillus rodentium* 11822_3, *Lactobacillus rodentium* 11822_4, *Lactobacillus rodentium* 11822_5 and *Lactobacillus rodentium* 11822_8 were grown in 4.5 ml of MRS broth at +37 °C for 48 hours. *Lactococcus lactis ssp. cremoris* 11809_6 was grown in 4.5 ml of M17+0.5% glucose.

The cultures were centrifugated 4000 rpm, 10 minutes, washed with sterile PBS, suspensed into sterile PBS pH 3 and incubated at room temperature for 2 hours. The bacterial cells were centrifugated 4000 rpm, minutes, washed with sterile PBS and suspensed into 1ml of 0.9% NaCl. Serially diluted samples were plated into MRS agar or M17 + 0.5% glucose agar and the plates were incubated 48h at +37 °C.

**Table 12. Bacterial counts (log cfu/ml) of pH 3 treated (2h, rt) bacterial cultures on MRS and M17 plates after 48 hours incubation**

| Strain | log CFU/ml |
|---|---|
| *Lactococcus lactis ssp. cremoris* 11809_6 | 8.04 |
| *Lactobacillus rodentium* 11822_3 | 7.98 |
| *Lactobacillus rodentium* 11822_4 | 6.77 |
| *Lactobacillus rodentium* 11822_5 | 7.99 |
| *Lactobacillus rodentium* 11822_8 | 8.16 |

### Conclusions

*Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822_8 tolerated the pH3 treatment for 2 hours which suggest that in probiotic application they have good potential to survive in acidic conditions of stomach.

### Example 8: Bile tolerance assay of lactic acid bacteria strains in vitro

Objective of the study was to evaluate the bile tolerance of lactic acid bacteria strains, *Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822_8.

### Methods and results

*Lactobacillus rodentium* 11822_3, *Lactobacillus rodentium* 11822_4, *Lactobacillus rodentium* 11822_5 and *Lactobacillus rodentium* 11822_8 were grown in 4.5 ml of MRS broth at +37 °C for 48 hours. *Lactococcus lactis ssp. cremoris* 11809_6 was grown in 4.5 ml of M17 + 0.5% glucose.

The bile resistance of *L. rodentium* 11822_3, *L. rodentium* 11822_4, *L. rodentium* 11822_5 and *L. rodentium* 11822_8 was analysed by culturing the strains in MRS-broth containing 0%, 0.3% or 1.0 % OxGall bile salts in 96-well plate for 48 hours. The bile resistance of *L. lactis ssp. cremoris* 11809_6 was analysed by culturing the strains in M17 + 0.5% glucose broth containing 0%, 0.3% or 1.0 % OxGall bile salts in 96-well plate for 48 hours. Enterococci and lactococci strains were incubated in aerobic conditions and lactobacilli in anaerobic environment.

**Table 13. Absorbance (570 nm) of the bacterial culture on MRS or M17 + 0.5% glucose broth containing 0%, 0.3% or 1.0% OxGall bile salts. Bacterial cultures were incubated at +37 °C for 48 hours.**

| Strain | Abs 570 nm | ABS 570 nm, 0.3% OxGall | ABS 570 nm, 1.0% OxGall |
|---|---|---|---|
| Control MRS aerobic | 0.204 | 0.213 | 0.223 |
| Control MRS anaerobic | 0.219 | 0.228 | 0.242 |
| *Lactobacillus rodentium* 11822_2 | 0.532 | 0.258 | 0.276 |
| *Lactobacillus rodentium* 11822_4 | 1.139 | 0.272 | 0.259 |
| *Lactobacillus rodentium* 11822_5 | 0.869 | 0.284 | 0.261 |
| *Lactobacillus rodentium* 11822_8 | 1.170 | 0.282 | 0.248 |
| Control M17 aerobic | 0.067 | 0.079 | 0.082 |
| *Lactococcus lactis ssp. cremoris* 11809_6 | 0.418 | 0.099 | 0.094 |

### Conclusions

*Lactococcus lactis ssp cremoris* 11809_6, *Lactobacillus rodentium* 11822_3, *Lactobacillus rodentium* 11822_4, *Lactobacillus rodentium* 11822_5 and *Lactobacillus rodentium* 11822_8 tolerated 0.3% and 1.0% bile concentrations, but growth was reduced compared to control.

### Example 9: Characterization of antibiotic susceptibility of identified strains

Objective of the study was to analyze the antibiotic susceptibility of gentamicin, kanamycin, streptomycin, tetracycline, erythromycin, clindamycin, chloramphenicol, ampicillin and vancomycin.

### Example 10: Characterization of antibiotic susceptibility of Lactococcus lactis ssp. cremoris 11809_6

### Methods and results

Antibiotic susceptibility of the strains *Lactococcus lactis* ssp. *cremoris* was analysed according to ISO10932:2010 standard (with necessary modifications) with VetMIC Lact-1 and VetMIC Lact-2 plates (SVA National Veterinary Institute, Uppsala, Sweden) in anaerobic conditions, at +37°C for 48 hours using LSM-medium (isosensitest broth: MRS broth 9:1).

Results of antibiotic susceptibility of strains are presented in Tables 2, 3 and 4. The minimum inhibitory concentrations (MIC) were compared with MIC cut-off values reported by EFSA (EFSA 2012. Guidance on the assessment of bacterial susceptibility to antimicrobials of human and veterinary importance. EFSA Journal 2012, 10(6), 2740).

Antibiotic susceptibility assay showed that none of the MIC-values for *Lactococcus lactis* ssp. *cremoris* 11809_6 exceeded the EFSA MIC cut-off values.

**Table 14. Minimum Inhibitory Concentration (MIC)-values of antibiotics required by European Food Safety Authority (EFSA) for Lactococcus lactis ssp. cremoris 11809_6.**

| ***Lactococcus lactis* ssp. *cremoris 11809_6*** | MIC µg/ml | EFSA MIC cut-off µg/ml |
|---|---|---|
| Gentamicin | 1 ₐ | 32 |
| Kanamycin | 8 | 64 |
| Streptomycin | 32_{b} | 32 |
| Tetracycline | 1 _{c} | 4 |
| Erythromycin | 0.12 | 1 |
| Clindamycin | 0.06_{d} | 1 |
| Chloramphenicol | 4 | 8 |
| Ampicillin | 0.25 | 2 |
| Vancomycin | 0.5 | 4 |

| | | |
|---|---|---|
| ₐ 2 µg/ml for one replicate, _{b} 16 µg/ml for one replicate, _{c} 0.5 µg/ml for one replicate _{d} 0.12 µg/ml for one replicate | | |

### Conclusions

The minimum inhibitory concentrations of the tested antibiotics for *Lactococcus lactis ssp. cremoris* 11809_6 did not exceed the MIC cut-off values proposed by EFSA.

### Summary of the results of the characterization of antibiotic susceptibility of identified strains

### Conclusion for 11809_6

The minimum inhibitory concentrations of the tested antibiotics for *Lactococcus lactis ssp. cremoris* 11809_6 did not exceed the MIC cut-off values proposed by EF-SA.

### Conclusion for 11822_3

The minimum inhibitory concentration of tetracycline for *Lactobacillus rodentium* 11822_3 exceeded EFSA MIC cut-off value, but because the value was only two times the cut-off value, the result is inside of tolerance limits and accepted by EFSA.

### Conclusion for 11822_4

The minimum inhibitory concentration of tetracycline for *Lactobacillus rodentium 11822_4* exceeded EFSA MIC cut-off value, but because the value was only two times the cut-off value, the result is inside of tolerance limits and accepted by EFSA.

### Conclusion for 11822_5

The minimum inhibitory concentration of tetracycline for *Lactobacillus rodentium* 11822_5 exceeded EFSA MIC cut-off value, but because the value was only two times the cut-off value, the result is inside of tolerance limits and accepted by EFSA.

### Conclusion for 11822_8

The minimum inhibitory concentration of tetracycline for *Lactobacillus rodentium* 11822_8 exceeded EFSA MIC cut-off value, but because the value was only two times the cut-off value, the result is inside of tolerance limits and accepted by EFSA.

### Example 11: Adhesion trial of lactic acid bacteria strains with C2BBe1 cell line

Objective of the study was to evaluate the adhesion properties of lactic acid bacteria strains *Lactococcus lactis ssp cremoris* 11809_6, *Lactobacillus rodentium* 11822-3, *Lactobacillus rodentium* 11822_4, *Lactobacillus rodentium* 11822_5 and *Lactobacillus rodentium* 11822_8.

### Methods and results

C2BBe1- enterocyte cells (Caco-2 clone, ATCC CRL-2102) were cultured in Dulbecco's modified Eagle medium (4500 mg/l glucose, 4.0mM L-glutamine, 110 mg/l sodium pyruvate, HyClone) supplemented with 10% Fetal Bovine Serum (HyClone), non essential amino acids (HyClone), NaHCO₃ (HyClone) and 10 mg/l human transferrin (Sigma). The cells were seeded into 24 well plate (2.5-3x10⁵ cells/well) and were cultured for 14 days in 1 ml medium volume. The medium replaced with fresh medium 3 times a week.

Meanwhile the bacterial cultures were prepared for adhesion trial so that the samples would be ready when the C2BBe1 cells were incubated for 14 days.

*Lactobacillus rhamnosus* GG was grown aerobically in 5 ml of MRS broth at +37 °C for 64 hours. Fresh MRS broth was inoculated with before mentioned culture (1%) and was incubated aerobically at +37 °C for 24 hours.

*Lactobacillus rodentium* 11822_3, *Lactobacillus rodentium* 11822_4, *Lactobacillus rodentium* 11822_5 and *Lactobacillus rodentium* 11822_8 were grown anaerobically in 5 ml of MRS broth at +37 °C for 64 hours. Fresh MRS broth was inoculated with before mentioned culture (1%) and was incubated anaerobically at +37 °C for 24 hours.

*Lactococcus lactis ssp. cremoris* 11809_6 was grown aerobically in M17 + 0.5% glucose at +37 °C for 64 hours. Fresh M17 broth+0.5% glucose was inoculated with before mentioned culture (1%) and was incubated aerobically at +37 °C for 24 hours.

The 24 hour incubated bacterial cultures were cetrifuged 4000 rpm for 10 minutes. The supernatant was removed and cells were washed 3x2ml sterile phosphate buffered saline (PBS). The cells were resuspened into 6 ml of C2BBe1-medium. The absorbance of the bacterial cells in C2BBe1-medium was adjusted with C2BBe1-medium to 1,4-1,6 (OD600nm). The bacterial counts of adjusted bacterial cultures were determined arerobically on M17+0.5 glucose agar *(Lactococcus lactis ssp cremoris* 11809_6), aerobically on MRS agar (*L. rhamnosus* GG) or anaerobically on MRS agar (*L. rodentium* 11822_3, *L. rodentium* 11822_4, *L. rodentium* 11822_5 and *L. rodentium* 11822_8).

The C2BBe1 cells , incubated for 14 days, were exposed to *Lc. lactis ssp cremoris* 11809_6, *L. rodentium* 11822_3, *L. rodentium* 11822_4, *L. rodentium* 11822_5 and *L. rodentium* 11822_8 cells for 1 hour at +37 °C. *L. rhamnosus* GG, the commercial strain which is known for good adhesion ability, was used as positive control. M17 broth+0.5% glucose and MRS broth were used as negative controls and C2BBe1-medium was used as zero control.

After the exposure, the medium was removed from the wells and cells were washed three times with 1ml PBS (prewarmed to +37 °C). The C2BBe1-cells were lysed with sterile filtered 0.1% Triton X-100 in 0.9% NaCl and incubated for 10 minutes in room temperature. Decimal dilutions were prepared from the samples. *Lc. lactis* ssp. *cremoris* 11809_6 and M17+ 0.5% glucose broth control samples were plated on M17+ 0.5% glucose agar and incubated aerobically at +37 °C for 48 hours. L. *rhamnosus* GG, background control and MRS broth control samples were plated on MRS agar and incubated aerobically at +37 °C for 48 hours. *L. rodentium* 11822_3, *L. rodentium* 11822_4, *L. rodentium* 11822_5 and *L. rodentium* 11822_8 samples were plated on MRS agar and incubated anaerobically at +37 °C for 48 hours.

### Results

**Table 15. Microbial counts of adhesion trial (log cfu/ml)**

| **Sample** | **Log cfu/ml before exposure** | **Mean log cfu/ml (n=3)** | **Standard deviation** |
|---|---|---|---|
| *Lactococcus lactis* ssp. *cremoris* 11809_6 | 8.81 | 7.40 | 0.15 |
| *Lactobacillus rodentium* 11822_3 | 7.18 | 6.66 | 0.13 |
| *Lactobacillus rodentium* 11822_4 | 7.18 | 7.03 | 0.14 |
| *Lactobacillus rodentium* 11822_5 | 7.18 | 6.93 | 0.27 |
| *Lactobacillus rodentium* 11822_8 | 7.18 | 6.68 | 0.24 |
| *Lactobacillus rhamnosus* GG | 8.47 | 6.87 | 0.13 |
| Broth control MRS broth | 0.00 | 0.00 | 0.00 |
| Broth control M17 + 0.5% glucose | 0.00 | 0.00 | 0.00 |
| Background control: C2BBe1-medium | 0.00 | 0.00 | 0.00 |

### Conclusions

*Lactococcus lactis* ssp. *cremoris* 11809_6, *Lactobacillus rodentium* 11822_3 *Lactobacillus rodentium* 11822_4, *Lactobacillus rodentium* 11822_5 and *Lactobacillus rodentium* 11822_8 were able to adhere on C2BBe1-cells at level equal or higher than the well known adherent strain *Lactobacillus rhamnosus* GG.

### Summary of the experimental part

After preliminary tests (results not shown here) the next important step was to define the antibiotic resistance of selected unidentified samples. The concentration of the antibiotics in test conditions was defined according to cut-off values of EFSA (2012). According to the test results 12 strains were sensitive to antibiotics. For these strains the additional antibiotic resistance tests (MIC tests) were performed, and according the results two strains were eliminated. The 16sRNA sequencing was done to ten strains to identify them. In this identification two *Enterococcus avium,* four *Lactobacillus rodentium,* three *Streptococcus gallolyticus* and one *Lactococcus lactis ssp cremoris* were identified. Streptococcus strains were eliminated, because their probiotic use might not be reasonable. For remaining strains MIC-tests (antibiotic susceptibility) were done according to EFSA rules, and also the ability to grow together with wellknown starter and in milk and the pH and bile tolerance were tested. Finally, the adhesion properties onto the intestinal cells was evaluated. According all these test results five bacteria strains were considered having potential as probiotic.

## Claims

1. A lactic acid bacteria strain for probiotic use **characterized by** 16 S RNA sequence defined by SEQ ID NO: 1 and having deposit number VTT E-173549.

2. A biologically active culture comprising a strain of at least one lactic acid bacteria for probiotic use **characterized by** 16 S RNA sequence defined by SEQ ID NO: 1 and having deposit number VTT E-173549.

3. The strain according to claim 1 or the culture according to claim 2, wherein the strain has an ability to adhere to mucosa in gastrointestinal tract.

4. The strain according to claim 1 or 3 or the culture according to claim 2 or 3, wherein the strain has a sugar usage profile shown in Table 1.

5. Feed composition or a nutritional supplement for canine, which comprises the strain according to claim 1 or the culture according to claim 2.

6. The composition according to claim 5, wherein said composition is a milk product, preferably fermented milk product.

7. The composition or the supplement according to claim 5 or 6, wherein said composition or supplement further comprises a starter culture.

8. The composition or the supplement according to any one of claims 5 to 7, wherein said composition or supplement is a feed for canine.

9. The composition or the supplement according to claim 7, wherein the bacteria are lyophilized or spray dried.

10. Lactic acid bacteria **characterized by** 16 S RNA sequence defined by SEQ ID NO: 1 and having deposit number VTT E-173549 for use in maintaining intestinal health and/or treatment and/or prophylactics of diarrhea.

11. Use of lactic acid bacteria **characterized by** 16 S RNA sequence defined by SEQ ID NO: 1 and having deposit number VTT E-173549 for preparation of feed composition or a nutritional supplement for canine.

12. A method for preparation of the composition according to claim 5 comprising the steps of
a) fat standardization; and
b) protein-standardization; and
c) homogenization; and
d) pasteurization; and
e) cooling; and
f) inoculation with starter cultures; and
g) incubation and cooling; and
h) stirring; and
i) pasteurization; and
j) inoculation with the strain according to claim 1 or a culture according to claim 2; and
k) packing.

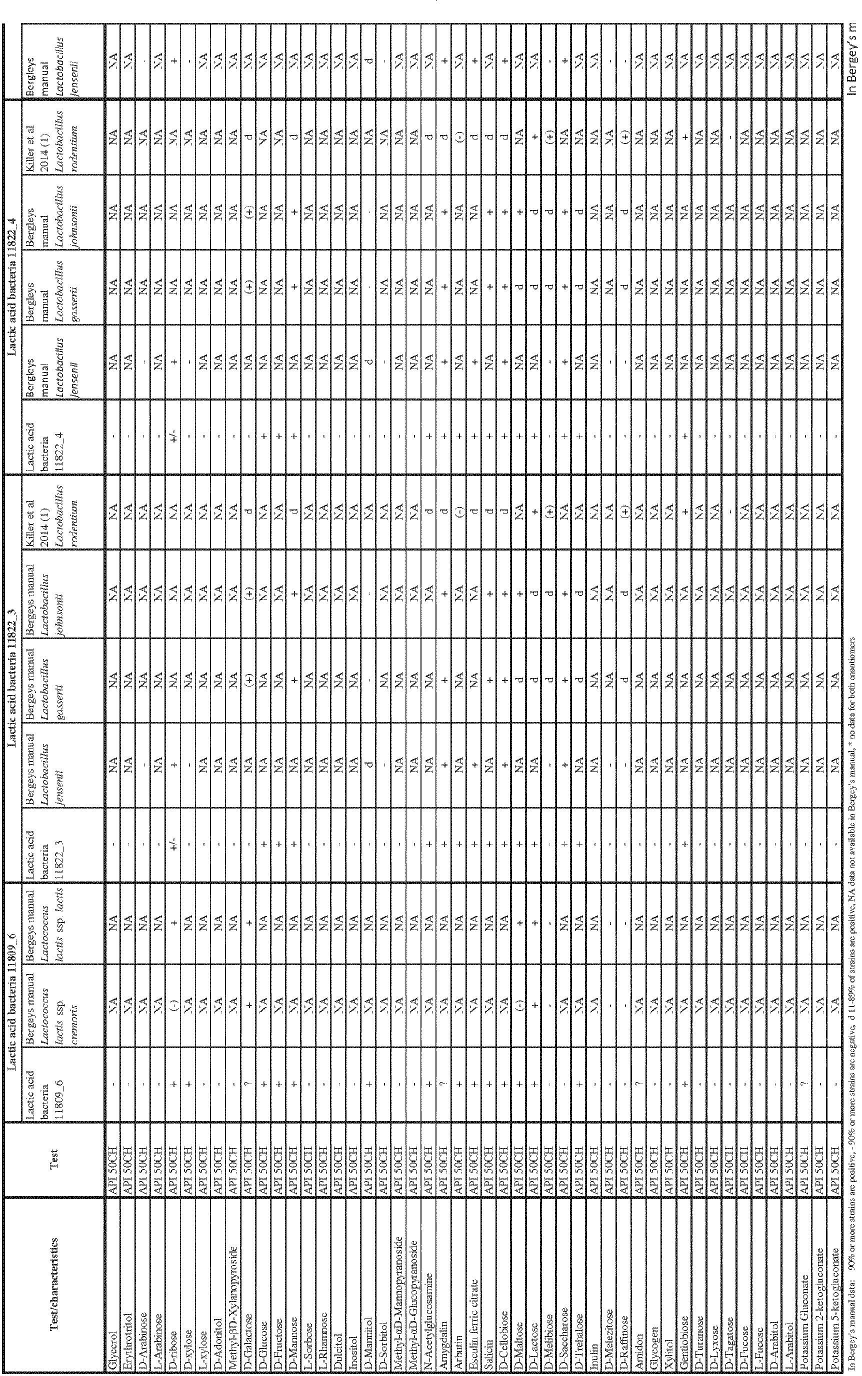

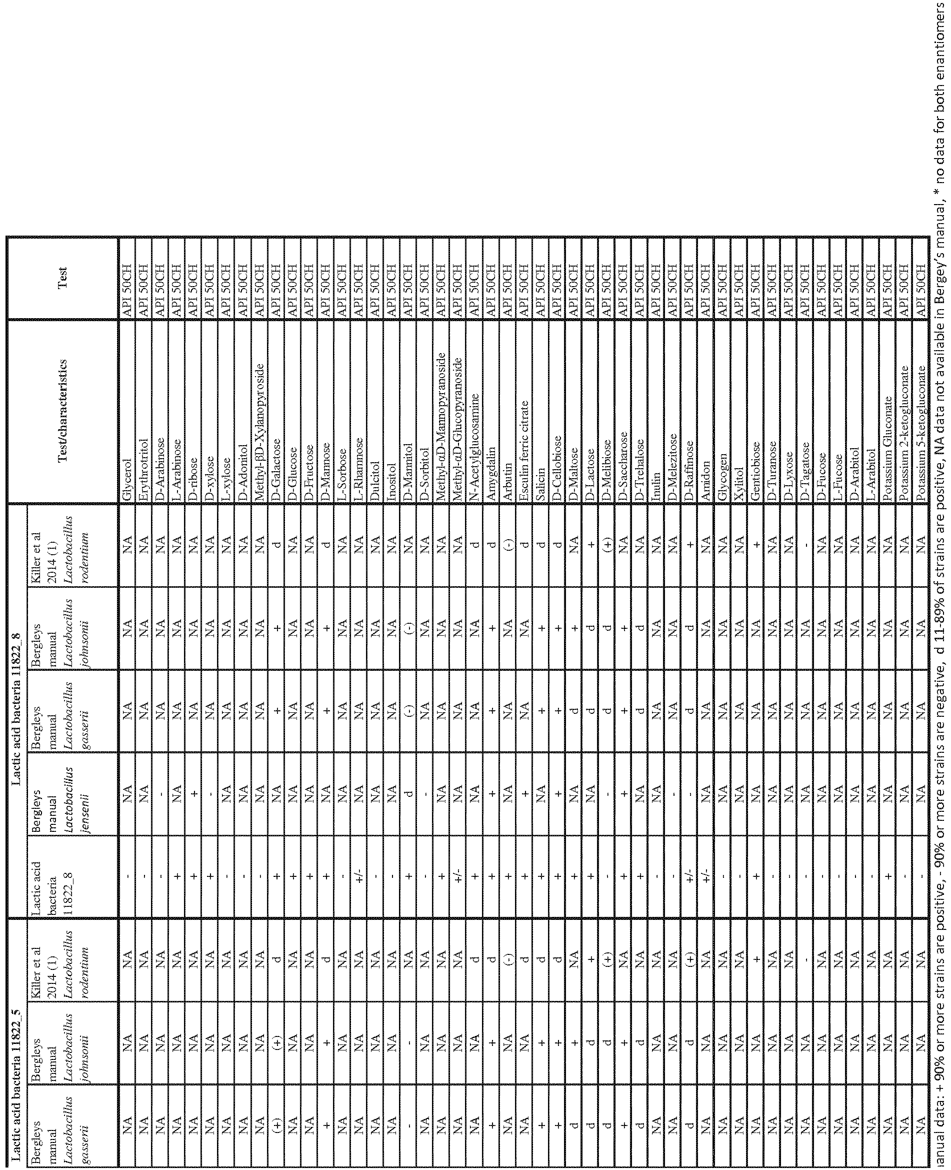

## Patentansprüche

1. Milchsäurebakterienstamm zur probiotischen Verwendung, der **gekennzeichnet ist durch** eine 16S-RNA-Sequenz, definiert durch SEQ ID NO: 1 und mit der Hinterlegungsnummer VTT E-173549.

2. Biologisch aktive Kultur, umfassend einen Stamm von mindestens einem Milchsäurebakterium zur probiotischen Verwendung, **gekennzeichnet durch** eine 16S-RNA-Sequenz, definiert durch SEQ ID NO: 1 und mit der Hinterlegungsnummer VTT E-173549.

3. Stamm gemäß Anspruch 1 oder Kultur gemäß Anspruch 2, wobei der Stamm die Fähigkeit hat, an der Schleimhaut im Magen-Darm-Trakt zu haften.

4. Stamm gemäß Anspruch 1 oder 3 oder Kultur gemäß Anspruch 2 oder 3, wobei der Stamm ein in Tabelle 1 gezeigtes Zuckerverwertungsprofil aufweist.

5. Futterzusammensetzung oder Nahrungsergänzungsmittel für Hunde, das den Stamm gemäß Anspruch 1 oder die Kultur gemäß Anspruch 2 umfasst.

6. Zusammensetzung gemäß Anspruch 5, wobei die Zusammensetzung ein Milchprodukt, vorzugsweise ein fermentiertes Milchprodukt ist.

7. Zusammensetzung oder Ergänzungsmittel gemäß Anspruch 5 oder 6, wobei die Zusammensetzung oder das Ergänzungsmittel ferner eine Starterkultur umfasst.

8. Zusammensetzung oder Ergänzungsmittel gemäß einem der Ansprüche 5 bis 7, wobei die Zusammensetzung oder das Ergänzungsmittel ein Futtermittel für Hunde ist.

9. Zusammensetzung oder Ergänzungsmittel gemäß Anspruch 7, wobei die Bakterien lyophilisiert oder sprühgetrocknet sind.

10. Milchsäurebakterien, **gekennzeichnet durch** eine 16S-RNA-Sequenz, definiert durch SEQ ID NO: 1 und mit der Hinterlegungsnummer VTT E-173549, zur Verwendung in der Aufrechterhaltung der Darmgesundheit und/oder der Behandlung und/oder Prophylaxe von Durchfall.

11. Verwendung von Milchsäurebakterien, die durch eine 16S-RNA-Sequenz, definiert durch SEQ ID NO: 1 und mit der Hinterlegungsnummer VTT E-173549, gekennzeichnet sind, zur Herstellung einer Futterzusammensetzung oder eines Nahrungsergänzungsmittels für Hunde.

12. Verfahren zur Herstellung der Zusammensetzung gemäß Anspruch 5, umfassend die folgenden Schritte
a) Fettstandardisierung; und
b) Proteinstandardisierung; und
c) Homogenisieren; und
d) Pasteurisieren; und
e) Abkühlen; und
f) Inokulation mit Starterkulturen; und
g) Inkubation und Abkühlen; und
h) Rühren; und
i) Pasteurisieren; und
j) Inokulation mit dem Stamm gemäß Anspruch 1 oder einer Kultur gemäß Anspruch 2; und
k) Verpacken.

## Revendications

1. Souche de bactérie lactique destinée à une utilisation probiotique, **caractérisée par** une séquence d'ARN 16S définie par SEQ ID n° 1 et ayant un numéro de dépôt VTT E-173549.

2. Culture biologiquement active, comprenant une souche d'au moins une bactérie lactique destinée à une utilisation probiotique, **caractérisée par** une séquence d'ARN 16S définie par SEQ ID n° 1 et ayant un numéro de dépôt VTT E-173549.

3. Souche selon la revendication 1 ou culture selon la revendication 2, la souche étant apte à adhérer aux muqueuses dans un tractus gastro-intestinal.

4. Souche selon la revendication 1 ou 3, ou culture selon la revendication 2 ou 3, la souche ayant un profil d'utilisation des sucres présenté dans le Tableau 1.

5. Composition d'aliment pour animaux ou complément nutritionnel pour les canidés, comprenant la souche selon la revendication 1 ou culture selon la revendication 2.

6. Composition selon la revendication 5, ladite composition étant un produit de lait, préférablement un produit de lait fermenté.

7. Composition ou complément selon la revendication 5 ou 6, ladite composition ou ledit complément comprenant en outre une culture starter.

8. Composition ou complément selon l'une quelconque des revendications 5 à 7, ladite composition ou ledit complément étant un aliment pour animaux de type canidé.

9. Composition ou complément selon la revendication 7, où les bactéries sont lyophilisées ou séchées par pulvérisation.

10. Bactérie lactique, **caractérisée par** une séquence d'ARN 16S définie par SEQ ID n° 1 et ayant un numéro de dépôt VTT E-173549, destinée à une utilisation dans le maintien de la santé des intestins et/ou le traitement et/la prophylaxie de la diarrhée.

11. Utilisation d'une bactérie lactique, **caractérisée par** une séquence d'ARN 16S définie par SEQ ID n° 1 et ayant un numéro de dépôt VTT E-173549, pour la préparation d'une composition d'aliment pour animaux ou d'un complément nutritionnel pour les canidés.

12. Méthode de préparation de la composition selon la revendication 5, comprenant les étapes consistant à :
a) une standardisation des matières grasses ; et
b) une standardisation des protéines ; et
c) une homogénéisation ; et
d) une pasteurisation ; et
e) un refroidissement ; et
f) une inoculation par des cultures starter ; et
g) une incubation et un refroidissement ; et
h) une agitation ; et
i) une pasteurisation ; et
j) une inoculation par la souche selon la revendication 1 ou une culture selon la revendication 2 ; et
k) un emballage.
